# EUROPÄISCHE PATENTSCHRIFT

(11) **EP 2 665 440 B1**
(45) Veröffentlichungstag und Bekanntmachung des Hinweises auf die Patenterteilung: **11.03.2020**
(21) Anmeldenummer: 12702461.0
(22) Anmeldetag: 20.01.2012
(51) Int. Cl.: A61F 2/18, A61F 2/86, A61N 1/05

(54) **HÖRPROTHESE**
HEARING PROSTHESIS
PROTHÈSE AUDITIVE

(30) Priorität: 20.01.2011 DE 102011009020
(43) Veröffentlichungstag der Anmeldung: 27.11.2013
(73) Patentinhaber: Acandis GmbH, 75177 Pforzheim (DE)
(72) Erfinder: CATTANEO, Giorgio, 76199 Karlsruhe (DE)
(74) Vertreter: Kilchert, Jochen
(86) Internationale Anmeldenummer: PCT/EP2012/050877
(87) Internationale Veröffentlichungsnummer: WO 2012/098232

(56) Entgegenhaltungen:
- EP-A1- 1 674 117
- EP-A1- 1 790 380
- WO-A1-2007/090655
- US-A1- 2004 019 364
- US-A1- 2007 142 879
- US-A1- 2010 023 102
- US-A1- 2010 087 905
- US-B1- 6 231 516
- US-B1- 7 515 966

## Beschreibung

Die Erfindung bezieht sich auf eine Hörprothese mit einem Innenohrimplantat. Ferner betrifft die Erfindung ein Verfahren zu Herstellung einer derartigen Hörprothese. Hörprothesen der eingangs genannten Art sind aus der Praxis bekannt.

Das Innenohr ist im Wesentlichen schneckenförmig ausgebildet und wird auch als Innenohrschnecke bzw. cochlea bezeichnet. Innerhalb der Innenohrschnecke befindet sich ein flüssiges Medium, die perilymphe, das die von außen über die Gehörknöchelchen in das Innenohr übertragenen akustischen Wellen im Innenohr verbreitet. Konkret ist das Innenohr aus zwei parallelen Kanälen gebildet, der scala vestibuli und der scala tympani, die an der Spitze, dem apex, der Innenohrschnecke durch eine Öffnung verbunden sind. Zwischen der scala vestibuli und der scala tympani erstreckt sich die scala media, die durch zwei Membrane von der scala tympani bzw. der scala vestibuli getrennt ist. In der scala media ist das corti-Organ angeordnet, das eine Vielzahl von Haarzellen umfasst. Die Haarzellen übertragen die Schwingungen der beiden Membrane, die die scala media von der scala vestibuli und der scala tympani trennen, in ein elektrisches Signal, das der Hörnerv, fachsprachlich der nervus vestibulocochlearis an das Gehirn übermittelt.

Die Haarzellen des corti-Organs nehmen entlang der cochlea bzw. der Innenohrschnecke unterschiedliche Frequenzen auf. Hochfrequente Schwingungen werden näher am Eingang der Innenohrschnecke von den Haarzellen erfasst, wogegen niederfrequente Schwingungen tiefer in die Innenohrschnecke eindringen und nahe des apex der Schnecke in elektrische Signale umgewandelt werden. Akustische Wellen mit niedriger Frequenz dringen also tiefer in das Innenohr ein als akustische Wellen mit höherer Frequenz.

Diese physiologische Besonderheit nutzen herkömmliche Hörprothesen, die mehrere auf einem Elektrodenträger angeordnete Elektroden umfassen. Der Elektrodenträger ist üblicherweise aus Kunststoff gebildet und weist eine schlauchartige Form auf. Auf der Außenfläche des Elektrodenträgers sind mehrere Elektroden angeordnet, die in Längsrichtung zueinander beabstandet sind. Im implantierten Zustand sind die Elektroden somit unterschiedlich tief im Innenohr bzw. der cochlea positioniert, so dass unterschiedliche Bereiche des Innenohrs elektrisch stimulierbar sind. Dadurch können verschiedene akustische Frequenzen nachgebildet werden.

Bekannte, herkömmliche Hörprothesen haben den Nachteil, dass der elektrische Kontakt zwischen den Elektroden und der Innenwand des Innenohrs nicht sicher gewährleistet werden kann, da die Innenohrstrukturen von Mensch zu Mensch unterschiedlich sind. Überdies ist die Anzahl der Elektroden auch dadurch begrenzt, dass die Zuleitungen zu den Elektroden, beispielsweise Kabel, die in dem Elektrodenträger angeordnet sind, den Durchmesser des Elektrodenträgers beeinflussen. Der Querschnittsdurchmesser des Elektrodenträgers begrenzt auch die Eindringtiefe des Innenohrimplantats bekannter Hörprothesen, so dass gerade Nervenzellen für tiefe Frequenzen schwer anregbar sind. Überdies erschwert die Steifigkeit bekannter Hörprothesen, insbesondere des Innenohrimplantats, die Implantation.

Aus anderen Bereichen der Medizintechnik sind bereits Elektroden bekannt, die zur dauerhaften Implantation geeignet sind. Insbesondere sind Elektroden zum Einsatz in Herz-Kreislauf-Systemen bekannt, wobei erreicht werden soll, elektrische Signale zur Therapie von Herz-Rhythmus-Störungen an die entsprechenden elektrischen Erregerzentren oder Reizleitungsbahnen des Herzens zu übertragen. Eine zu diesem Zweck geeignete Elektrode ist beispielsweise in US 5,531,779 offenbart.

Die bekannte Elektrode ist zur Übertragung relativ hoher Ströme ausgelegt, die zur Behandlung von Herzfehlern erforderlich sind. Die Elektrode ist als endovaskulärer Stent ausgebildet, der aus einer Stahllegierung hergestellt und insgesamt leitfähig ist. Der gesamte Stent bildet damit eine Sonde, die zur Einfuhr in den Körper komprimierbar und expandierbar ist. Zur Erzeugung eines elektrischen Feldes wird die Stentelektrode mit einer weiteren Elektrode kombiniert, die in Form einer zweiten Stentelektrode ausgebildet ist, die von der ersten Stentelektrode beabstandet implantiert und unterschiedlich polarisiert wird.

Die bekannte Stentelektrode ist aus einzelnen Drähten aufgebaut, die im expandierten Zustand im Wesentlichen parallel verlaufen und korbförmig ausgebildet sind. Andere auf Drähten basierende Stentstrukturen, wie beispielsweise gewebte oder geflochtene Stents werden als geeignet offenbart. Das bekannte System ermöglicht nur eine unpräzise und schlecht modulierbare Stimulation größerer Körperbereiche.

Eine ähnliche Elektrode ist aus US 2003/74039 A1 bekannt, die ebenfalls zur Herzbehandlung eingesetzt wird. Die Elektrode wird mit einer weiteren zusätzlichen Elektrode verwendet, um ein elektrisches Feld zu erzeugen.

Ferner sind Elektroden bekannt, bei denen die Elektrodenpole in ein und demselben Implantat integriert sind, sodass die zweite separate Elektrode entfallen kann. US 6,445,953 B1 offenbart beispielsweise eine Stentelektrode, an deren Außenfläche eine erste und zweite Elektrode befestigt sind, die durch ein Funksignal angesteuert werden. Ein weiteres Beispiel für eine implantierbare Elektrode, bei der die beiden Pole im selben Implantat angeordnet sind, ist aus WO 2008/094344 A1 bekannt, die einen spiralförmigen Draht offenbart, an welchem verschiedene Elektrodenbereiche bzw. Pole vorgesehen sind. Die verschiedenen Elektrodenbereiche können unabhängig voneinander angesteuert werden, so dass das mit der Elektrode erzeugbare elektrische Feld modulierbar ist. Eine ähnliche Elektrode ist aus WO2008/094789 A1 bekannt, die zur Verankerung des Elektrodendrahtes eine Stentstruktur aufweist. Diese ist mit dem Elektrodendraht verbunden, sodass durch die Expansion des Trägerstents der Elektrodendraht an die Gefäßwand gepresst wird. Der Stent dient dabei ausschließlich als mechanische Stütze. Eine weitere Elektrode aus einem Draht mit unterschiedlichen polarisierbaren Elektrodenbereichen ist aus EP 1 304 135 A2 bekannt.

Die Herstellung der vorstehend genannten Elektroden ist kompliziert, da hierfür entweder aufwändige Fügetechniken notwendig sind oder die Stromversorgung der einzelnen Elektrodenbereiche schwierig zu realisieren ist. Außerdem sind die mit der Stützstruktur verbundenen Elektroden verhältnismäßig groß, sodass einerseits die Anzahl der Elektroden limitiert ist und andererseits eine präzise Modulation der elektrischen Felder nur eingeschränkt möglich ist. Für einige der bekannten Systeme sind Leitungen erforderlich, die mit der Elektrode verbunden sind und entsprechend isoliert sein müssen, da andernfalls ein Kontakt mit der tragenden Struktur der Elektrode zu einem Kurzschluss führen würde. Die isolierten Leitungen erhöhen die Komplexität und Sperrigkeit der Systeme und erschweren die Zufuhr, insbesondere in kleine Gefäße.

Die vorgenannten Elektroden, die für die Verwendung im Herz-Kreislauf-System vorgesehen sind, unterscheiden sich von Elektroden bekannter Hörprothesen insbesondere durch die Eigenschaft, einen komprimierten Zustand und einen expandierten Zustand einzunehmen, und eignen sich nicht für die Stimulation in der cochlea, da die erzeugbaren elektrischen Felder unpräzise und schlecht modulierbar sind. Elektroden für Hörprothesen sind um Unterschied zu den gitterartig strukturieren Elektroden für Herz-Kreislauf-Behandlungen üblicherweise starr bzw. auf einen Elektrodenträger angeordnet, der einen unveränderbaren Querschnitt aufweist. Die Einstellung eines guten elektrischen Kontakts zwischen den Elektroden und der Innenwand des Innenohrs wird somit erschwert. Die Implantation bekannter Hörprothesen ist daher relativ aufwändig.

EP 1 790 380 A1 offenbart eine mehrschichtige Mikroelektrode für den Einsatz in medizinischen Implantaten, insbesondere auch Cochlear-Implantaten, und insofern eine Hörprothese gemäß dem Oberbegriff des Patentanspruchs 1.

Der Erfindung liegt die Aufgabe zugrunde, eine Hörprothese mit einem Innenohrimplantat anzugeben, die einfach implantierbar ist sowie eine verbesserte Anregung ermöglicht.

Erfindungsgemäß wird die Aufgabe im Hinblick auf die Hörprothese durch den Gegenstand des Anspruchs 1 gelöst.

Die Erfindung beruht auf dem Gedanken, eine Hörprothese mit einem Innenohrimplantat anzugeben, das eine Tragstruktur und wenigstens zwei in Längsrichtung voneinander beabstandet angeordnete Elektroden umfasst. Die Elektroden sind getrennt voneinander aktivierbar und mit der Tragstruktur verbunden. Die Tragstruktur ist stentartig komprimierbar und expandierbar, so dass die Tragstruktur an die Form des Innenohrs anpassbar ist.

Der Erfindung liegt die Idee zu Grunde, eine Tragstruktur, insbesondere einen Elektrodenträger, zu nutzen, die sich flexibel an die Geometrie, insbesondere die Querschnittsgeometrie, der scala tympani oder scala vestibuli anpassen kann. Daher ist erfindungsgemäß vorgesehen, dass die Tragstruktur stentartig komprimierbar und expandierbar ist. Ähnlich wie ein Stent, der in ein Blutgefäß einsetzbar ist und eine radiale Aufweitung des Blutgefäßes bewirkt, ist mit der erfindungsgemäßen Hörprothese beabsichtigt, durch die radial expandierbare Tragstruktur einen sicheren Kontakt oder zumindest einen geringen Abstand, also eine größere Nähe, zwischen den Elektroden und der Innenwand des Innenohrs, insbesondere der scala tympani, zu erreichen. Dadurch wird einen verbesserte elektrische Signalübertragung in das corti-Organ bewirkt. Insbesondere erfolgt die elektrische Signalübertragung über die Basilarmembran, die die scala tympani von der scala media trennt, in das corti-Organ. Die stentartig komprimierbare Tragstruktur erleichtert zudem die Implantation, da die Tragstruktur bzw. allgemein das Innenohrimplantat im komprimierten Zustand einfach und mit vergleichsweise geringem Verletzungsrisiko in das Innenohr, insbesondere die cochlea, einbringbar ist. Die Expansion der Tragstruktur erfolgt vorzugsweise im Anschluss an die vollständige Positionierung der Tragstruktur bzw. des Innenohrimplantats in der scala tympani. Die stentartige bzw. stentartig komprimierbare und expandierbare Tragstruktur ist insgesamt flexibel, so dass sich das Innenohrimplantat bei der Implantation gut an die schneckenförmige Geometrie der cochlea anpasst. Die Komprimierbarkeit der Tragstruktur erhöht auch die Zugänglichkeit des Innenohrimplantats in tiefer gelegene Abschnitte der cochlea, da das Innenohrimplantat mit der komprimierten Tragstruktur beispielsweise über miniaturisierte Kathetersysteme weit in die Innenohrschnecke eingeführt werden kann. Somit ermöglicht die erfindungsgemäße Hörprothese auch die Anregung von Haarzellen bzw. Nervenbahnen in der Nähe des apex der cochlea, so dass auch besonders tiefe Schallfrequenzen übertragbar sind. Die Hörleistung wird damit insgesamt verbessert. Die Hörprothese kann die Hörfunktion, insbesondere die Funktion des Außenohrs, Mittelohrs und teilweise des Innenohrs, unter Anwendung technischer Mittel künstlich ersetzen oder zumindest unterstützen.

Erfindungsgemäß sind wenigstens zwei Elektroden vorgesehen, die in Längsrichtung beabstandet angeordnet sind. Dies bietet die Voraussetzung dafür, unterschiedlich tief in der cochlea angeordnete Abschnitte des corti-Organs elektrisch zu stimulieren, so dass verschieden hohe bzw. tiefe Frequenzen anregbar sind. Die Längsrichtung des Innenohrimplantats bzw. der Tragstruktur folgt im implantierten Zustand dem schneckenartigen Verlauf der cochlea bzw. des Innenohrs.

Das Innenohrimplantat mit der stentartig komprimierbaren und expandierbaren Tragstruktur bietet weitere Vorteile, beispielsweise bei der Implantation des Innenohrimplantats bei Kindern. Durch die radial flexible Struktur des Innenohrimplantats, insbesondere der Tragstruktur, kann sich das Innenohrimplantat an eine sich wachstumsbedingt vergrößernde cochlea anpassen. Die erfindungsgemäße Hörprothese wächst also mit. Zusätzliche Anpassungsoperationen, wobei größere Innenohrimplantate eingesetzt werden, können somit vermieden werden. Durch die Anpassungsfähigkeit der Tragstruktur wird auch bei Veränderung des Innenohrs, beispielsweise durch Wachstum, ein gleichbleibender elektrischer Kontakt zwischen den Elektroden und dem corti-Organ sichergestellt. Eine konstante und gleichbleibende Stimulationsleistung ist die Folge.

Bei einer bevorzugten Ausführungsform der erfindungsgemäßen Hörprothese weist die Tragstruktur eine Gitterstruktur aus Gitterstegen oder ein Gittergeflecht oder eine coilartige Spiralstruktur auf. Die unterschiedlichen Gestaltungsformen der Tragstruktur haben unterschiedliche Vorteile und können je nach Einsatzzweck vom Fachmann entsprechend gewählt werden. Eine coilartige Struktur ist besonders einfach herstellbar und weist im komprimierten Zustand einen äußerst kleinen Querschnittsdurchmesser auf. Beispielsweise kann die coilartige Struktur im komprimierten Zustand im Wesentlichen einem länglichen Draht entsprechen, der sich bei der Expansion spiralförmig aufweitet bzw. eine Spiralform einnimmt. Die coilartige Struktur zeigt sich dabei erst im expandierten Zustand. Somit ermöglicht eine derartige Tragstruktur eine besonders tiefe Implantation in der cochlea.

Gitterstrukturen zeichnen sich durch eine verbesserte radiale Stabilität aus. Insbesondere können Gitterstrukturen im Allgemeinen eine erhöhte Radialkraft aufweisen, so dass eine bündige Anlage der Tragstruktur an die Innenwand des Innenohrs sichergestellt ist. Überdies können Gitterstrukturen im Allgemeinen eine vergleichsweise große Außenoberfläche bilden, die mit dem Innenohr in Kontakt ist. Vorteilhaft können als Gitterstruktur ausgebildete Tragstrukturen eine hohe Anzahl an Elektroden tragen. Gitterstrukturen aus geflochtenen Drähten, also Gittergeflechte, sind zudem sehr flexibel und weisen eine vorteilhafte Anpassungsfähigkeit an die schneckenartige Krümmung der cochlea auf. Gitterstrukturen aus Gitterstegen, also Gitterstrukturen, die im Wesentlichen einstückig aus einem Vollmaterial gebildet sind, weisen vorzugsweise eine Außenfläche auf, die sich im Wesentlichen in einer Ebene erstreckt, so dass die Auflagefläche zur Innenwand des Innenohrs einheitlich ist. Überdies bietet das Herstellungsverfahren von Gitterstrukturen aus Gitterstegen Vorteile hinsichtlich der Anordnung der Elektroden auf bzw. in der Tragstruktur.

Die Herstellung einer Gitterstruktur aus Gitterstegen erfolgt beispielsweise durch Ätzen oder Schneiden aus einem Vollmaterial. Die Öffnungen, die die Zellen der Gitterstruktur bilden, werden dabei aus dem Vollmaterial herausgeschnitten bzw. geätzt, so dass die die Zellen begrenzenden Gitterstege übrigbleiben. Alternativ dazu kann die Herstellung einer auf Gitterstegen basierenden Gitterstruktur durch ein Abscheideverfahren, beispielsweise ein physikalisches Abscheideverfahren (PVD-Verfahren) erfolgen. Bei einem Abscheideverfahren wird das Material der Gitterstege auf einem Substrat abgeschieden, wobei die gesamte Gitterstruktur im Wesentlichen schichtweise aufgebaut wird. Dieses Herstellungsverfahren eignet sich besonders zur Herstellung freitragender Gitterstrukturen. Es ist auch möglich, dass eine bestehende, aus einem Vollmaterial hergestellte Gitterstruktur durch ein Abscheideverfahren beschichtet wird, um besondere Eigenschaften, insbesondere leitende Eigenschaften, der Tragstruktur einzustellen. Im Allgemeinen kann die auf Gitterstegen basierende Gitterstruktur zunächst flach hergestellt, beispielsweise abgeschieden, werden und anschließend in eine Rohrform bzw. rotationssymmetrische Form überführt werden. Ein Verfahren zur Herstellung einer flachen Gitterstruktur ist beispielsweise in DE 10 2006 039 840 A1 offenbart, die auf die Anmelderin zurückgeht. Es ist aber auch möglich, die Gitterstruktur bereits in einer Rohrform bzw. rotationssymmetrischen Form direkt herzustellen. In diesem Zusammenhang wird darauf hingewiesen, dass sich die rotationssymmetrische bzw. röhrchenartige Form im Wesentlichen im expandierten, unbelasteten Zustand der Gitterstruktur bzw. allgemein Tragstruktur zeigt. Im implantierten Zustand, wenn die Tragstruktur an der Innenwand des Innenohrs anliegt, passt sich die Querschnittsgeometrie der Tragstruktur der Querschnittsgeometrie des Innenohrs, insbesondere der scala tympani an. Somit bildet die Tragstruktur im implantierten Zustand eine Rohrform, die nicht zwingend einen kreisförmigen Querschnitt, sondern einen Querschnitt aufweist, der an die Form des Innenohrs angepasst ist.

Die Elektroden des Innenohrimplantats sind vorzugsweise stoffschlüssig mit der Tragstruktur verbunden, insbesondere in die Tragstruktur integriert. Die Verbindung zwischen den Elektroden und der Tragstruktur ist somit besonders stabil, so dass ein Ablösen der Elektroden verhindert ist. Überdies ist die Herstellung des Innenohrimplantats bzw. allgemein der Hörprothese durch die in die Tragstruktur integrierten bzw. stoffschlüssig mit der Tragstruktur verbundenen Elektroden vereinfacht, da ein zusätzlicher Fügeschritt eingespart wird. Vielmehr werden die Elektroden vorzugsweise einteilig mit der Tragstruktur ausgebildet. Mit anderen Worten kann die Tragstruktur zumindest bereichsweise die Elektroden bilden.

Erfindungsgemäß ist vorgesehen, dass die Tragstruktur einen mehrschichtigen Aufbau mit wenigstens einer elektrisch leitenden äußeren Modulationsschicht und wenigstens einer elektrisch isolierten Isolationsschicht aufweist. Die Modulationsschicht bildet wenigstens eine Elektrode. Die Isolationsschicht ist unterhalb der äußeren Modulationsschicht angeordnet. Der mehrschichtige Aufbau der Tragstruktur ermöglicht eine weitere Miniaturisierung, wodurch die Komprimierbarkeit der Tragstruktur verbessert und somit die Implantation des Innenohrimplantats erleichtert wird. Insbesondere ermöglicht der mehrschichtige Aufbau eine verbesserte Zugänglichkeit des Innenohrimplantats zu tiefer liegenden Abschnitten des Innenohrs. Die Modulationsschicht bildet wenigstens eine Elektrode und ist daher vorzugsweise auf einer Außenfläche bzw. einer Außenumfangsfläche der Tragstruktur angeordnet. Die Isolationsschicht ist hingegen radial nach innen versetzt bzw. unter der Modulationsschicht angeordnet. Die Isolationsschicht und die Modulationsschicht sind vorzugsweise direkt miteinander verbunden. Für die Herstellung einer Tragstruktur mit dem mehrschichtigen Aufbau eignet sich insbesondere ein Abscheideverfahren, wobei äußerst dünne Schichten herstellbar sind, so dass insgesamt die Komprimierbarkeit der Tragstruktur verbessert ist.

Die äußere Modulationsschicht kann zumindest bereichsweise eine Außenfläche der Tragstruktur bilden und durch die Isolationsschicht unterbrochen sein, so dass die Isolationsschicht und die äußere Modulationsschicht zumindest in Längsrichtung abwechselnd angeordnet sind. Dabei können die durch die äußere Modulationsschicht gebildeten Elektroden durch die Isolationsschicht voneinander elektrisch isoliert und separat aktivierbar sein. Bei dieser Variante bildet die äußere Modulationsschicht also beide bzw. allgemein mehrere Elektroden, die voneinander elektrisch isoliert sind. Dazu ist vorgesehen, dass die Modulationsschicht zumindest in Längsrichtung der Tragstruktur bzw. des Innenohrimplantats durch die Isolationsschicht unterbrochen ist. Die Tragstruktur kann also mehrere Bereiche aufweisen, die die Außenoberfläche der Tragstruktur bilden, wobei wenigstens ein Bereich elektrisch leitend durch die Modulationsschicht und ein weiterer Bereich elektrisch isoliert durch die Isolationsschicht gebildet ist. Es können mehrere voneinander isolierte elektrische Bereiche bzw. Bereiche der äußeren Modulationsschicht vorgesehen sein, die getrennt voneinander ansteuerbar bzw. aktivierbar sind. Das hat den Vorteil, dass mit einer, insbesondere einer einzigen Modulationsschicht mehrere separat aktivierbare Elektroden gebildet werden können, so dass die Wandstärke der Tragstruktur bzw. allgemein des Innenohrimplantats klein ist. Die Komprimierbarkeit wird dadurch verbessert und die Implantation des Innenohrimplantats erleichtert.

Erfindungsgemäß ist eine elektrisch leitende innere Modulationsschicht vorgesehen. Die innere Modulationsschicht ist durch die Isolationsschicht von der äußeren Modulationsschicht elektrisch isoliert. Dabei bildet die äußere Modulationsschicht zumindest bereichsweise eine Außenfläche, insbesondere eine Außenoberfläche, der Tragstruktur. Die äußere Modulationsschicht und die Isolationsschicht sind bereichsweise unterbrochen, so dass die innere Modulationsschicht freiliegt bzw. bereichsweise die Außenfläche bildet. Die äußere Modulationsschicht und die innere Modulationsschicht sind in Längsrichtung des Innenohrimplantats bzw. der Tragstruktur abwechselnd angeordnet, so dass die innere Modulationsschicht und die äußere Modulationsschicht jeweils wenigstens eine separat aktivierbare Elektrode bilden. Konkret sind die nach außen freiliegenden Oberflächen, also die Außenoberflächen, der beiden Modulationsschichten abwechselnd angeordnet.

Somit wechseln sich die elektrisch wirksamen Bereiche der Modulationsschichten entlang des Innenohrimplantats ab.

Der mehrschichtige Aufbau der Tragstruktur kann insgesamt mehrere Modulationsschichten umfassen, die jeweils durch eine Isolationsschicht voneinander getrennt sind. Dabei können die unterschiedlichen Modulationsschichten, insbesondere die äußere Modulationsschicht und die innere Modulationsschicht jeweils wenigstens eine Elektrode bilden. Dazu ist vorgesehen, dass die äußere Modulationsschicht und die Isolationsschicht, die die äußere Modulationsschicht von der inneren Modulationsschicht isoliert, bereichsweise unterbrochen ist, so dass die innere Modulationsschicht freigelegt ist. Mit anderen Worten wird die Außenoberfläche der Tragstruktur bei dieser Variante einerseits durch die äußere Modulationsschicht und andererseits durch die innere Modulationsschicht gebildet, wobei die unterschiedlichen Modulationsschichten jeweils eine separat aktivierbare Elektrode bilden. Die freigelegten Bereiche, also die Bereiche, in denen die Außenoberfläche der Tragstruktur durch die innere Modulationsschicht gebildet ist, sind mit den Bereichen, in denen die äußere Modulationsschicht die Außenfläche der Tragstruktur bildet, in Längsrichtung des Innenohrimplantats abwechselnd angeordnet. Damit ist sichergestellt, dass sich die unterschiedlich bzw. separat aktivierbaren Elektroden in Längsrichtung des Innenohrimplantats abwechseln, um unterschiedliche Bereiche des Innenohrs in Abhängigkeit der jeweiligen Schallfrequenz zu simulieren.

Die beiden vorgenannten Varianten, nämlich die teilweise unterbrochene Modulationsschicht, die auf diese Weise mehrere Elektroden bildet, und der mehrschichtige Aufbau der Tragstruktur aus mehreren Modulationsschichten, können miteinander kombiniert werden. Vorteilhaft weist die Tragstruktur also einen mehrschichtigen Aufbau mit einer äußeren Modulationsschicht und wenigstens einer inneren Modulationsschicht auf, wobei die äußere Modulationsschicht und die innere Modulationsschicht jeweils unterbrochen sind und durch die Isolationsschicht voneinander getrennte elektrische Bereiche, die Elektroden, bilden, so dass sowohl in der äußeren Modulationsschicht, als auch in der inneren Modulationsschicht mehrere separat aktivierbare Elektroden ausgebildet sind. Auf diese Weise kann die Elektrodendichte entlang des Innenohrimplantats erhöht werden, ohne die Komprimierbarkeit der Tragstruktur erheblich zu beeinflussen. Insbesondere im Vergleich zu einer Tragstruktur mit einer einzigen Modulationsschicht kann die Anzahl der Elektroden erhöht werden. Insgesamt ist somit eine engere Abstufung des stimulierbaren Frequenzbandes möglich, was die Hörleistung bzw. die Genauigkeit des Hörerlebnisses für den Patienten steigert.

Die Elektroden sind vorzugsweise jeweils durch ein stromleitendes Element mit einer Stromversorgung gekoppelt. Das stromleitende Element kann ein Kabel, das entlang der Tragstruktur geführt ist, oder eine Leitungsbahn umfassen, die in die Tragstruktur integriert ist. Um einen Kurzschluss zu vermeiden, sind die Kabel vorzugsweise isoliert. Die Kabel können lose auf der Tragstruktur angeordnet sein, oder mit der Tragstruktur, insbesondere den Gitterstegen einer Gitterstruktur oder den Drähten eines Gittergeflechts verbunden sein. Durch die Verbindung der Kabel mit der Tragstruktur wird der kontrollierte Verlauf der Kabel begünstigt. Die Kabel können sich über die Tragstruktur hinaus erstrecken, um die Elektroden mit einer entfernten Stromversorgung bzw. Steuereinheit zu verbinden.

Bei einer bevorzugten Ausführungsform der erfindungsgemäßen Hörprothese ist die Leitungsbahn durch die äußere Modulationsschicht oder die innere Modulationsschicht gebildet. Bei einem mehrschichtigen Aufbau der Tragstruktur mit wenigstens der äußeren Modulationsschicht und der Isolationsschicht ist es vorteilhaft, wenn die Modulationsschicht nicht nur die Elektroden, sondern auch die Leitungsbahn bzw. allgemein das stromleitende Element bildet. Im Allgemeinen kann die Tragstruktur also nach Art einer Elektronikplatine aufgebaut sein, wobei innerhalb des mehrschichtigen Aufbaus der Tragstruktur mehrere Leitungsbahnen gebildet sind, die die jeweiligen Elektroden mit der Stromversorgung verbinden. Die Leitungsbahnen können sich zumindest bis zu einem proximalen Ende der Tragstruktur erstrecken und beispielsweise mit Kabeln verbunden sein oder in Kabel übergehen, die die Strecke von der Stromversorgung bis zur Tragstruktur überbrücken. Bei dieser Variante ist also das stromleitende Element in die Tragstruktur integriert, so dass insgesamt ein kompaktes Innenohrimplantat bereitgestellt wird.

Die äußere Modulationsschicht und/oder die innere Modulationsschicht können stellenweise unterbrochen sein, so dass die äußere Modulationsschicht und/oder die innere Modulationsschicht jeweils mehrere Leitungsbahnen und Elektroden bilden. Die Elektroden der äußeren Modulationsschicht und die Elektroden der inneren Modulationsschicht sind vorzugsweise jeweils separat aktivierbar. Wie zuvor bereits erläutert, kann die Tragstruktur also einen mehrschichtigen Aufbau mit wenigstens zwei Modulationsschichten, einer äußeren Modulationsschicht und wenigstens einer inneren Modulationsschicht, aufweisen, wobei die Modulationsschichten jeweils innerhalb der Schicht unterbrochen sind, um innerhalb einer der Modulationsschichten mehrere separat aktivierbare bzw. voneinander isolierte elektrisch leitende Bereiche zu schaffen. Die separaten elektrischen Bereiche einer einzigen Modulationsschicht können somit unterschiedliche Elektroden bilden. Gleichzeitig können die separaten elektrisch leitenden Bereiche einer Modulationsschicht die jeweilige Leitungsbahn für eine separat aktivierbare Elektrode bilden. Somit können bei einer im Wesentlichen rohrförmigen Tragstruktur auf dem Umfang mehrere Leitungsbahnen nebeneinander in einer einzigen Schicht bzw. in einer einzigen Ebene angeordnet sein. Gleichzeitig können mehrere Leitungsbahnen in radialer Richtung übereinander angeordnet sein, so dass sich durch den mehrschichtigen Aufbau in Kombination mit mehreren in einer Schicht angeordneten Leitungsbahnen eine erhöhte Anzahl von Leitungsbahnen bzw. stromleitenden Elementen ergibt, die in die Tragstruktur integriert sind. Die Anzahl der separat ansteuerbaren Elektroden ist somit erhöht.

Bei einer weiteren bevorzugten Ausführungsform der erfindungsgemäßen Hörprothese ist das Innenohrimplantat mit einer Steuereinheit, insbesondere einem Sprachprozessor, verbunden oder verbindbar, so dass die Elektroden zur Modulation elektrischer Felder variabel ansteuerbar sind. Dabei kann die Steuereinheit eine implantierbare Empfangsspule umfassen, die mit einer externen Sendespule der Steuereinheit drahtlos gekoppelt ist. Durch die drahtlose Kopplung zwischen der Sendespule und der Empfangsspule kann einerseits Energie und andererseits ein Steuersignal übermittelt werden, das die unterschiedliche Ansteuerung verschiedener Elektroden bewirkt. Die Energieübertragung kann beispielsweise induktiv erfolgen, so dass in der internen implantierten Empfangsspule ein Strom induziert wird, der zur Stimulation entsprechender Bereiche des corti-Organs an die jeweilige Elektrode geleitet wird. Die Steuereinheit kann hierzu insbesondere einen Sprachprozessor umfassen, der vorzugsweise extern angeordnet und mit der Sendespule verbunden ist. Der Sprachprozessor setzt die empfangenen akustischen Signale in elektrische Signale um und sendet das Steuersignal für die entsprechenden, anzusteuernden Elektroden aus. Der Sprachprozessor kann programmierbar bzw. einstellbar sein, so dass das Hörempfinden individuell an den Patienten bzw. die Lage der Elektroden im Innenohr angepasst werden kann.

Vorzugsweise ist die Steuereinheit, insbesondere der Sprachprozessor mit den Elektroden derart signalverbunden, dass unterschiedliche Elektroden in Abhängigkeit von der jeweiligen Schallfrequenz aktivierbar sind. Die Steuereinheit, insbesondere der Sprachprozessor, ist also derart angepasst, dass eine Ansteuerung einzelner Elektroden oder einer Elektrodengruppe in Abhängigkeit der Schallfrequenz der empfangenen akustischen Signale erfolgt. Insbesondere ist vorgesehen, dass Elektroden, die im implantierten Zustand des Innenohrimplantats näher am apex der Innenohrschnecke angeordnet sind, angesteuert werden, wenn Schallwellen mit tiefer Frequenz empfangen werden. Beim Empfang von Schallwellen mit höherer Frequenz werden vorzugsweise die Elektroden oder Elektrodengruppen im vorderen Bereich, insbesondere in der Nähe des Eingangsbereichs der Innenohrschnecke aktiviert.

Das Innenohrimplantat kann mehrere Elektroden umfassen, die einerseits in Längsrichtung und andererseits im Umfangsrichtung der Tragstruktur angeordnet sind. Für die Stimulation unterschiedlicher Bereiche des corti-Organs zur Simulation unterschiedlicher Hörfrequenzen ist es vorteilhaft, wenn die Elektroden in Längsrichtung des Innenohrimplantats beabstandet voneinander angeordnet sind. Zusätzlich können in Umfangsrichtung des Innenohrimplantats mehrere Elektroden angeordnet sein, so dass jeweils die in Umfangsrichtung angeordnete Elektrode aktivierbar ist, die dem corti-Organ im implantierten Zustand am nächsten liegt. Positionierungsungenauigkeiten bei der Implantation können somit ausgeglichen und die Hörleistung verbessert werden. Ferner ermöglicht die Anordnung der Elektroden zusätzlich in Umfangsrichtung eine verbesserte Modulation elektrischer Felder, da beispielsweise zwei in Umfangsrichtung benachbart angeordnete Elektroden zur Erzeugung eines elektrischen Feldes miteinander verschaltbar sind. Somit werden die Möglichkeiten zur Erzeugung unterschiedlicher elektrischer Felder, die unterschiedliche Teilbereiche des corti-Organs gezielt stimulieren, erhöht. Das durch die Hörprothese abgedeckte Frequenzspektrum wird somit verbessert.

Die Hörprothese kann mit einem transkutanen Energieübertragungssystem ausgestattet sein. Die Energie kann somit drahtlos in den implantierbaren Teil der Hörprothese übertragen werden, so dass der implantierbare Teil, insbesondere das Innenohrimplantat gut als Dauerimplantat genutzt werden kann. Ein zusätzlicher operativer Eingriff, beispielsweise für einen Batteriewechsel, ist nicht erforderlich. Der implantierbare Teil der Hörprothese kann auch einen von extern aufladbaren Akkumulator oder alternativ eine auswechselbare Batterie umfassen.

Gemäß einem nebengeordneten Aspekt beruht die Erfindung auf dem Gedanken, ein Verfahren zur Herstellung einer zuvor beschriebenen Hörprothese anzugeben, bei dem die Tragstruktur zumindest teilweise durch physikalische Gasphasenabscheidung hergestellt wird. Dabei werden verschieden Materialien auf einem Substrat schichtweise abgeschieden und derart strukturiert, dass eine freitragende Tragstruktur oder eine beschichtete Tragstruktur mit wenigstens einer Modulationsschicht und wenigstens einer Isolationsschicht gebildet wird. Vorzugsweise wird die Tragstruktur in Form einer Gitterstruktur mit Gitterstegen abgeschieden. Der Einsatz von PVD-Verfahren, insbesondere von Sputterverfahren, zur Herstellung der Hörprothese, ermöglicht die Ausbildung von Gitterstegen mit verschiedenen Schichten aus unterschiedlichen Materialien. Somit können gezielt wenigstens eine Isolierschicht und wenigstens eine elektrisch leitende Modulationsschicht aufgebracht und entlang der Tragstruktur modifiziert werden. Auf diese Weise können nahezu beliebig angeordnete elektrisch leitende Bereiche bzw. Schichten und elektrisch isolierte Bereiche bzw. Schichten ausgebildet werden. Das erfindungsgemäße Verfahren ermöglicht eine sehr flexible Herstellung des Innenohrimplantats der Hörprothese, wobei die unterschiedlichen Bereiche bzw. Schichten der Tragstruktur je nach Anforderung an die Hörprothese angeordnet und im Hinblick auf die geometrische Form der Bereiche ausgebildet werden können. Andere Herstellungsverfahren sind möglich, bei denen die Isolationsschicht oder Modulationsschicht anderweitig auf die Tragstruktur aufgebracht wird. Beispielsweise können Fotolithographieverfahren zum Einsatz kommen.

Die Erfindung wird nachfolgend anhand von Ausführungsbeispielen unter Bezugnahme auf die beigefügten, schematischen Zeichnungen näher mit weiteren Einzelheiten erläutert. In diesen zeigen:
- Fig. 1: eine schematische, teilgeöffnete Darstellung einer Innenohrschnecke bzw. cochlea;
- Fig. 2: eine Querschnittsansicht der cochlea gemäß Fig. 1;
- Fig. 3: einen Querschnitt der cochlea gemäß Fig. 1, wobei das Innenohrimplantat der erfindungsgemäßen Hörprothese implantiert ist und Feldlinien des von den Elektroden erzeugten elektrischen Feldes eingezeichnet sind;
- Fig. 4: einen Längsschnitt durch das Innenohrimplantat gemäß Fig. 3;
- Fig. 5a: eine Draufsicht auf die Tragstruktur des Innenohrimplantats einer erfindungsgemäßen Hörprothese nach einem bevorzugten Ausführungsbeispiel mit drei in Längsrichtung beabstandet angeordneten Elektroden, die jeweils durch ein Kabel mit einer Stromversorgung gekoppelt sind;
- Fig. 5b: einen Längsschnitt durch die Tragstruktur gemäß Fig. 5a, wobei das elektrische Feld jeweils zwischen einer Elektrode und einer gemeinsamen, außerhalb des Innenohrimplantats angeordneten, Neutralelektrode erzeugt wird;
- Fig. 6a: eine Draufsicht auf eine Tragstruktur eines Innenohrimplantats der erfindungsgemäßen Hörprothese nach einem weiteren bevorzugten Ausführungsbeispiel, wobei sechs durch Kabel mit einer Stromversorgung verbundene Elektroden in Längsrichtung des Innenohrimplantats beabstandet angeordnet sind;
- Fig. 6b: einen Längsschnitt durch die Tragstruktur gemäß Fig. 6a, wobei jeweils zwei Elektroden zur Erzeugung eines lokalen elektrischen Feldes miteinander verschaltet sind;
- Fig. 7: eine Draufsicht auf die Tragstruktur des Innenohrimplantats einer erfindungsgemäßen Hörprothese nach einem weiteren bevorzugten Ausführungsbeispiel, wobei sowohl in Längsrichtung, als auch in Umfangsrichtung zueinander beabstandet angeordnete Elektroden vorgesehen sind;
- Fig. 8: einen Längsschnitt durch die Tragstruktur gemäß Fig. 5a, 6a oder 7;
- Fig. 9: zwei Ansichten eines Gitterstegs einer Tragstruktur eines Innenohrimplantats der erfindungsgemäßen Hörprothese mit einem mehrschichtigen Aufbau, wobei die durch die äußere Modulationsschicht gebildete Elektrode plättchenartig ausgebildet ist und sich über die Breite des Gitterstegs hinaus erstreckt;
- Fig. 10: zwei Ansichten des Gitterstegs gemäß Fig. 9 mit einer Querschnittsdarstellung außerhalb der plättchenartigen Elektrode;
- Fig. 11: eine Draufsicht auf die Tragstruktur gemäß Fig. 5a mit nach außen isolierten Leitungsbahnen, die in die Tragstruktur integriert sind;
- Fig. 12: einen Längsschnitt durch die Tragstruktur des Innenohrimplantats der Hörprothese nach einem erfindungsgemäßen Ausführungsbeispiel im implantierten Zustand;
- Fig. 13a: eine Draufsicht auf eine Tragstruktur der Hörprothese nach einem weiteren erfindungsgemäßen Ausführungsbeispiel, wobei die Tragstruktur eine Gitterstruktur aufweist;
- Fig. 13b: einen Ausschnitt der Gitterstruktur gemäß Fig. 13a; und
- Fig. 13c: eine Querschnittsansicht der Gitterstruktur gemäß Fig. 13b mit mehreren Modulationsschichten.

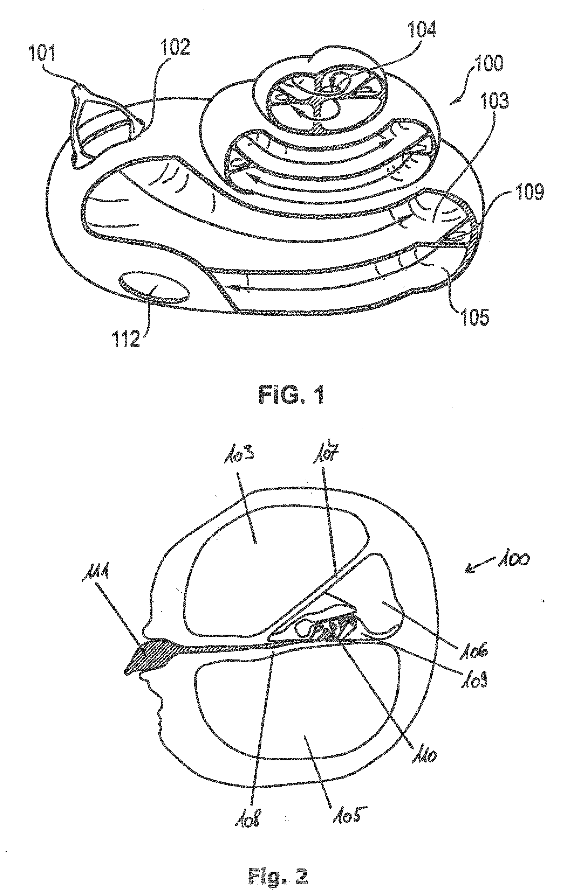

Die Fig. 1 und 2 zeigen den physiologischen Aufbau der Hörschnecke bzw. Innenohrschnecke, fachsprachlich cochlea. Die cochlea 100 weist ein ovales Fenster 102 auf, in das der Steigbügel 101 der Mittelohrknöchelchen eingreift. Über das ovale Fenster 102 überträgt der Steigbügel 101 akustische Schwingungen in das Innenohr, konkret in die scala vestibuli 103. Die Schwingungen werden über die in der scala vestibuli 103 eingeschlossenen perilymphe entlang der schneckenförmigen Windungen in Richtung des apex, also der Spitze, der cochlea 100 weitergeleitet. Die perilymphe füllt auch die scala tympani 105, die im Wesentlichen parallel zur scala vestibuli 103 verläuft und im Bereich des apex durch eine Öffnung, dem helicotrema 104, verbunden ist. Der Druckausgleich im Innenohr erfolgt über das runde Fenster 112 am Ende der scala tympani 105. Zwischen der scala vestibuli 103 und der scala tympani 105 ist die scala media 106 angeordnet und durch die Reissner-Membran 107 einerseits von der scala vestibuli 103 und durch die Basilarmembran 108 andererseits von der scala tympani 105 getrennt. In der scala media 106 verläuft das Corti-Organ 109, das eine Vielzahl von Haarzellen 110 umfasst. Die Haarzellen 110 sind mit dem nervus vestibulocochlearis 111 verbunden. Je nach Schallfrequenz, die in das Innenohr eingekoppelt wird, werden unterschiedliche Bereiche des Corti-Organs 109 entlang der Innenohrschnecke stimuliert. Abhängig von der Lage der stimulierten Haarzellen 110 werden unterschiedliche elektrische Signale an das Gehirn geleitet, so dass die unterschiedlichen Schallfrequenzen voneinander unterscheidbar sind. Dabei sind die Haarzellen 110 in der Nähe des apex der cochlea 100 für die Erkennung niedriger Frequenzen vorgesehen, wogegen die Haarzellen im vorderen Bereich, also in der Nähe des ovalen Fensters 102 hohe Schallfrequenzen in elektrische Signale umwandeln.

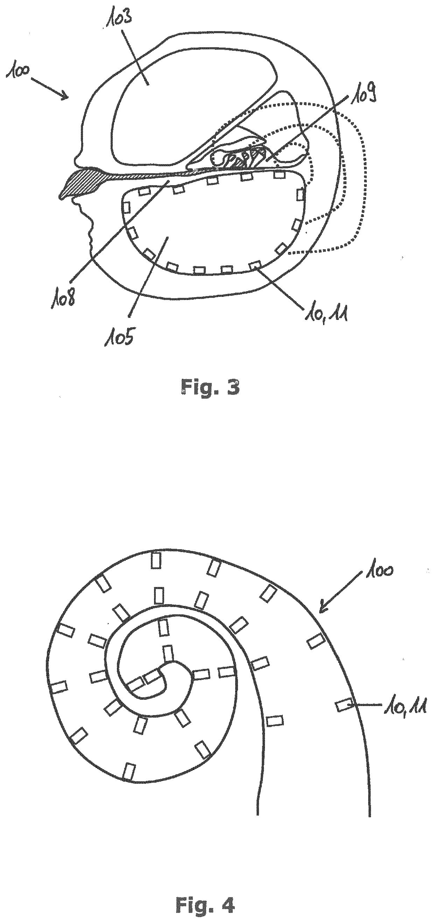

In Fig. 3 ist das Grundprinzip der erfindungsgemäßen Hörprothese im implantierten Zustand dargestellt. Die Hörprothese umfasst ein Innenohrimplantat 10, das eine Tragstruktur 11 und wenigstens zwei in Längsrichtung voneinander beabstandet angeordnete Elektroden 12 umfasst. Die Tragstruktur 11 ist stentartig komprimierbar und expandierbar. Vorzugsweise ist die Tragstruktur 11 aus einer Gitterstruktur gebildet, wobei besonders bevorzugt eine Gitterstruktur aus Gitterstegen zum Einsatz kommt. Wie in Fig. 3 gut erkennbar ist, passt sich die Gitterstruktur bzw. allgemein die Tragstruktur 11 des Innenohrimplantats im implantierten Zustand an die Innenwand des Innenohrs, konkret der scala tympani an, so dass ein guter Kontakt zwischen den Elektroden 12 und der cochlea 100 hergestellt ist. Vorzugsweise ist die Tragstruktur 11 an die Form des Innenohrs bzw. der cochlea 100 anpassbar derart, dass sich die Tragstruktur 11 an eine Innenwand des Innenohrs zumindest bereichsweise anlegt, insbesondere bündig anlegt. Ideal ist ein vollständiges Anlegen des Innenohrimplantats 10 an eine Innenwand der cochlea 100. Dabei besteht vorzugsweise zwischen allen Elektroden des Innenohrimplantats 10 und der Innenwand der cochlea 100 ein Kontakt. Es ist aber auch möglich, dass Bereiche des Innenohrimplantats 10 nicht an der Innenwand des Innenohrs anliegen. Beispielsweise kann das Innenohrimplantat 10 derart expandierbar sein, dass ein distaler Abschnitt des Innenohrimplantats 10 im expandierten Zustand an der Innenwand der cochlea 100 anliegt, wogegen ein proximaler Abschnitt des Innenohrimplantats 10 im expandierten Zustand einen Abstand zur Innenwand des Innenohrs aufweist. In jedem Fall bewirkt die Expansion des Innenohrimplantats 10 bzw. der Tragstruktur 11 eine starke Annäherung der Elektroden 12 an die Haarzellen des Innenohrs, so dass eine gute Stimulation ermöglicht ist.

Die Tragstruktur 11 kann zumindest abschnittsweise überdimensioniert sein derart, dass die Tragstruktur 11 im vollständig expandierten Zustand einen größeren Querschnittsdurchmesser als das Innenohr, insbesondere die scala tympani oder scala vestibuli aufweist. Damit wird erreicht, dass im implantierten Zustand die Tragstruktur 11 eine Restkraft auf die Innenwand des Innenohrs ausübt, so dass das Innenohrimplantat 10 insgesamt in seiner Position gehalten wird. Alternativ kann vorgesehen sein, dass die Tragstruktur 11 unterdimensioniert ist, also im vollständig expandierten Zustand einen kleineren Querschnittsdurchmesser als das Innenohr, insbesondere die scala tympani oder scala vestibuli, aufweist. Im implantierten Zustand legt sich die Tragstruktur 11 somit nicht vollumfänglich an die Innenwand des Innenohrs an. Dennoch wird durch die Expansion gewährleistet, dass sich die Elektroden an die Haarzellen ausreichend annähern, um eine gute Stimulation der Haarzellen zu erreichen. Insgesamt ermöglicht die expandiere und komprimierbare Tragstruktur 11 die Zufuhr des Innenohrimplantats 10 über einen Katheter, insbesondere Mikrokatheter, so dass die Implantation mikrochirurgisch durchführbar ist. Ferner ermöglicht die expandierbare Tragstruktur 11 eine erhöhte Flexibilität des Innenohrimplantats 10, so dass sich das Innenohrimplantat 10 gut an die Krümmung des Innenohrs anpassen kann. Das Innenohrimplantat 10 kann sowohl an die scala tympani angepasst sein, als auch in der scala vestibuli zum Einsatz kommen.

Die Anordnung der Tragstruktur 11 des Innenohrimplantats 10 ist im Längsschnitt gemäß Fig. 4 gut sichtbar. Darin ist zu sehen, dass die Tragstruktur 11 im implantierten Zustand, also nach der Überführung von einem komprimierten Zustand, in dem die Tragstruktur 11 in das Innenohr eingebracht wird, in einen expandierten Implantationszustand, weit in die cochlea 100 hineinragt, so dass auch die für den Empfang tiefer Frequenzen zuständigen Haarzellen 110 stimulierbar sind.

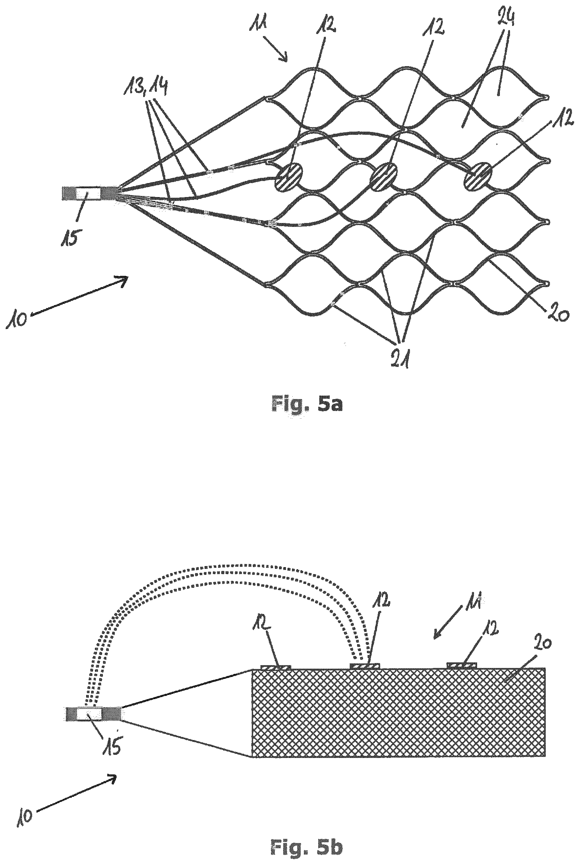

Nachfolgend wird auf die konstruktive Gestaltung des Innenohrimplantats 10 näher eingegangen:
Das Innenohrimplantat 10 umfasst die Tragstruktur 11, die mit den Elektroden 12 verbunden ist. Die Elektroden 12 können auf der Tragstruktur angeordnet sein und durch eine Fügetechnik mit der Tragstruktur 11 verbunden werden. In Fig. 5a sind die Elektroden 12 plättchenförmig ausgebildet und mit der Tragstruktur 11 verbunden, die als Gitterstruktur 20 ausgebildet ist. Die Tragstruktur 11 ist vorzugsweise als Gitterstruktur 20 aus Gitterstegen 21 gebildet, wobei die Gitterstege 21 im Wesentlichen in derselben Wandungsebene der Tragstruktur 11 angeordnet und in Kreuzungspunkten miteinander verbunden sind. In Fig. 5a ist ebenfalls erkennbar, dass die Elektroden 12, hier drei Elektroden 12, in Längsrichtung des Innenohrimplantats 10 beabstandet angeordnet sind. Dadurch wird ermöglicht, dass im implantierten Zustand unterschiedlich tief in der cochlea 100 liegende Bereiche des corti-Organs 109 stimulierbar sind.

Insgesamt weist die Tragstruktur 11 eine stentartige Struktur, also im Wesentlichen eine Gitterstruktur, auf. Die Gitterstruktur 20 kann als Open-Cell-Design ausgeführt sein. Die Gitterstruktur 20 weist also Zellen auf, die im Wesentlichen offen sind. Einzelne Umfangssegmente der Gitterstruktur 20 sind dabei durch Verbinder miteinander gekoppelt. Durch die Verbinder zwischen den Umfangssegmenten wird erreicht, dass die Gitterstruktur 20 bei der Expansion sich nicht nur radial aufweitet, sondern auch in Längsrichtung streckt. Somit kann sich die Länge des Innenohrimplantats 10 ändern. Das Open-Cell-Design ist besonders vorteilhaft bei der Verwendung der Hörprothese bei Kindern einsetzbar, da auf diese Weise ein besonders gutes Mitwachsen des Innenohrimplantats 10 gewährleistet ist. Das Innenohrimplantat 10 folgt nicht nur der Durchmesseränderung der cochlea im Wachstum, sondern auch der Längenänderung.

Alternativ kann die Tragstruktur 11, insbesondere die Gitterstruktur 20, im Closed-Cell-Design ausgeführt sein. Derartige Gitterstrukturen 20 haben den Vorteil, dass sie einen besonders kleinen komprimierten Querschnittsdurchmesser aufweisen und somit über miniaturisierte Kathetersysteme zuführbar sind. Überdies ermöglichen Closed-Cell-Desings das Wiedereinziehen in den Katheter nach erfolgter Expansion. Somit kann das Innenohrimplantat 10 bei einer Fehlpositionierung wieder in das Kathetersystem zurückgeführt und im gleichen Operationsvorgang repositioniert werden.

Des Weiteren ist es vorteilhaft, wenn der Tragstruktur 11 bereits bei der Herstellung die Spiralform des Innenohrs aufgeprägt wird. Dies ist eine bevorzugte Ausführungsform. Die Tragstruktur 11 kann derart ausgebildet sein, dass die Tragstruktur 11 im vollständig expandierten Zustand, also ohne äußere Krafteinwirkung, insbesondere im Herstellzustand, selbsttätig eine Spiralform bzw. Schneckenform einnimmt. Bei der Implantation wickelt sich die spiralförmig bzw. schneckenförmig vorgeformte Tragstruktur 11 selbsttätig auf, wobei die Tragstruktur 11 bzw. allgemein das Innenohrimplantat 10 der Spiralform bzw. Schneckenform der cochlea 100 folgt. Das Aufprägen der Innenohrstruktur auf die Tragstruktur 11 ist besonders einfach möglich, wenn die Tragstruktur 11, wie bei einem bevorzugten Ausführungsbeispiel vorgesehen, ein Formgedächtnismaterial umfasst. Durch Temperatureinwirkung nimmt das Formgedächtnismaterial der Tragstruktur 11 die ursprünglich aufgeprägte Form wieder ein. Die Implantation des Innenohrimplantats wird somit erleichtert.

Die Elektroden 12 sind mit einer Stromversorgung (nicht dargestellt) elektrisch verbunden. Insbesondere sind die Elektroden 12 jeweils durch ein stromleitendes Element 13 mit einer Stromversorgung gekoppelt. Das stromleitende Element 13 kann, wie in Fig. 5a dargestellt, ein isoliertes Kabel umfassen. Konkret sind die Elektroden 12 gemäß Fig. 5a jeweils durch ein separates isoliertes Kabel mit einer Stromversorgung kontaktiert. Die Kabel 14 können gemäß Fig. 5a lose auf der Tragstruktur 11 aufliegen bzw. angeordnet sein. Alternativ kann vorgesehen sein, dass die Kabel 14 mit den Gitterstegen 21 der Gitterstruktur 20 der Tragstruktur 11 verbunden sind. Letztere Variante hat den Vorteil, dass die Verbindung der Kabel 14 mit den Gitterstegen 21 den kontrollierten Verlauf der Kabel 14 begünstigt. Insbesondere ist eine Verbindung zwischen den Kabel 14 und der Tragstruktur 11 bei der Expansion der Tragstruktur 11 vorteilhaft. Wie in Figur 5a erkennbar ist, sind die Elektroden 12 in Längsrichtung der Tragstruktur 11 auf einer Linie angeordnet, die parallel zur Längsachse der Tragstruktur 11 verläuft. Alternativ kann vorgesehen sein, dass die Elektroden 12 spiralförmig um die Längsachse der Tragstruktur 11 angeordnet sind. Andere Anordnungen, beispielsweise eine musterartige Anordnung von Elektroden 12, ist möglich. Beispielsweise können die Elektroden 12 in Längsrichtung der Tragstruktur 11 versetzt, insbesondere zickzackförmig versetzt, angeordnet sein.

Fig. 5b verdeutlicht die axiale Anordnung der Elektroden 12 des Innenohrimplantats 10 gemäß Fig. 5a. Bei einer derartigen Anordnung der Elektroden 12 ist es vorteilhaft, das Innenohrimplantat 10 derart in die cochlea 100 zu implantieren, dass die Elektroden 12 an der Basilarmembran 108 anliegen, so dass die erzeugten elektrischen Felder im corti-Organ 109 auf die Haarzellen 110 bzw. den nervus vestibucochlearis 111 wirken. Die elektrischen Felder können dabei unterschiedlich erzeugt werden. In Fig. 5b ist beispielsweise dargestellt, dass die elektrischen Felder zwischen zwei Polen erzeugt werden, wobei einer der elektrischen Pole durch eine Elektrode 12 und ein weiterer der elektrischen Pole durch eine Neutralelektrode 15 erzeugt werden, wobei die Neutralelektrode 15 außerhalb der Tragstruktur 11 angeordnet ist. Beispielsweise kann die Neutralelektrode 15 am proximalen Ende der Tragstruktur 11, also im implantierten Zustand näher im Bereich des Mittelohrs oder Außenohrs angeordnet sein.

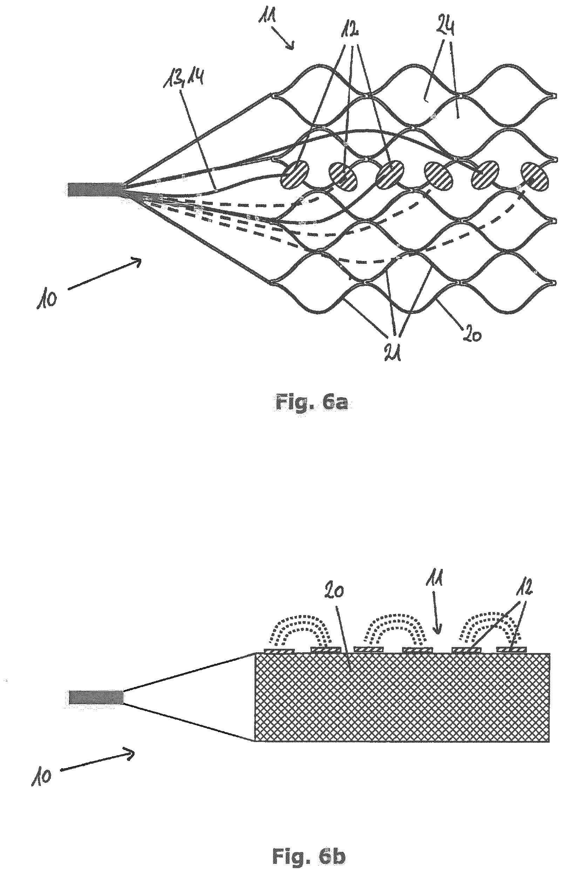

Alternativ kann vorgesehen sein, dass elektrische Felder erzeugt werden, die jeweils zwei benachbarte Elektroden 12 als Pole nutzen. Eine derartige Konfiguration ist in Fig. 6b gezeigt. Im Allgemeinen sind unterschiedliche Feldlinienformen bzw. elektrische Felder einstellbar. Die Elektroden 12 sind generell separat aktivierbar, so dass durch eine entsprechende Steuereinheit unterschiedliche elektrische Felder durch variable Verschaltung unterschiedliche Elektroden 12 und/oder wenigstens einer Neutralelektrode 15 erzeugbar sind. Dabei kann die Neutralelektrode auch außerhalb der cochlea 100 implantiert oder vollständig außerhalb des Körpers angeordnet sein. Beispielsweise kann die Neutralelektrode 15 auch im Bereich der Empfangsspule einer Steuereinheit angeordnet sein, wobei die Empfangsspule der Steuereinheit ebenfalls implantiert ist. Eine vereinfachte Konstruktion bietet die Anordnung einer Neutralelektrode 15 am proximalen Ende der Tragstruktur 11, beispielsweise an einer Stelle, an der die stromleitenden Elemente 13, insbesondere die Kabel 14, zusammengeführt sind.

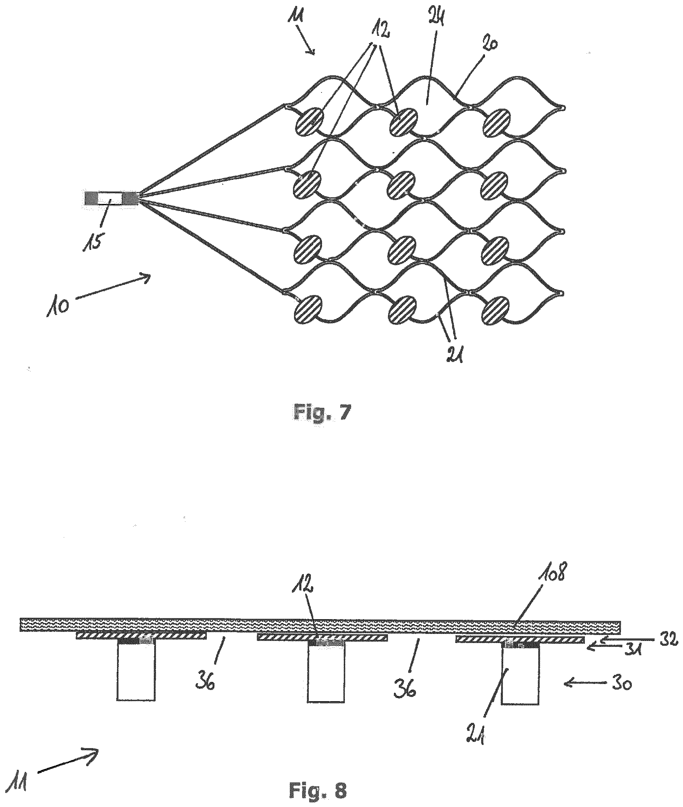

Wenn das elektrische Feld nicht zwischen einer Neutralelektrode 15 und einer weiteren Elektrode 12 erzeugt werden soll, sondern zwischen jeweils zwei Elektroden 12, die mit der Tragstruktur 11 verbunden sind, ist eine höhere Anzahl an Elektroden 12 auf der Tragstruktur 11 zweckmäßig. Eine derartige Konfiguration ist in Fig. 6a dargestellt, wobei insgesamt sechs Elektroden 12 in Längsrichtung des Innenohrimplantats 10 beabstandet zueinander angeordnet sind. Jeweils zwei Elektroden 12 bilden ein Elektrodenpaar, das zur Erzeugung eines elektrischen Feldes entsprechend ansteuerbar ist. Die Feldlinien der durch Elektrodenpaare erzeugbaren elektrischen Felder sind in Fig. 6b eingezeichnet. Die in Figur 6b dargestellten Feldlinien zeigen die bevorzugte Ausbreitung der erzeugten elektrischen Felder, wobei vorzugsweise jeweils zwischen zwei benachbart angeordneten Elektroden 12 ein elektrisches Feld erzeugt wird. Das elektrische Feld kann sich aber auch über mehrere Elektroden ausbreiten. Insbesondere kann sich das elektrische Feld auch in Umfangsrichtung zwischen zwei Elektroden ausbreiten, wie beispielsweise in Figur 3 dargestellt. Figur 7 zeigt ein Ausführungsbeispiel eines Innenohrimplantats 10, wobei die Elektroden 12 nicht nur in Längsrichtung benachbart zueinander angeordnet sind, sondern auch in Umfangsrichtung. Die zwischen den Elektroden 12 entstehenden elektrischen Felder können sich sowohl in Längsrichtung zwischen zwei Elektroden 12, als auch in Umfangsrichtung zwischen zwei Elektroden 12 oder schräg, also spiralförmig um die Längsachse des Innenohrimplantats 10, zwischen zwei Elektroden 12 ausbreiten. Dabei können die elektrischen Felder einzelne Elektroden 12 überspringen.

Für alle Ausführungsbeispiele gilt, dass mehrere Elektroden 12 entlang des Innenohrimplantats 10 angeordnet sein können. Insbesondere kann das Innenohrimplantat 10 12, 16, 20, 28, 36 oder 48 Elektroden 12 umfassen. Die Elektroden 12 können sich generell über den gesamten Umfang der Tragstruktur 11 erstrecken. Die Tragstruktur 11 kann also ringförmige Elektroden 12 umfassen, die den gesamten Umfang der Tragstruktur 11 umgreifen und in Längsrichtung der Tragstruktur 11 voneinander getrennt sind. Es ist auch möglich, dass die Elektroden 12 den Umfang der Tragstruktur 11 nur abschnitts- bzw. bereichsweise ausfüllen. In Umfangsrichtung der Tragstruktur 11 kann also teilweise eine Elektrode 12 vorgesehen sein. Der restliche Teil des Umfangsegments der Tragstruktur 11 kann nach außen isoliert sein.

Hinsichtlich der Tragstruktur 11 ist vorgesehen, dass diese aus einem Vollmaterial beispielsweise durch Laserschneiden hergestellt sein kann. Alternativ kann die Tragstruktur 11 eine geflochtene Gitterstruktur 20 bzw. ein Gittergeflecht umfassen oder coilartig gestaltet sein. Eine coilartige Tragstruktur 11 umfasst beispielsweise einen einzigen Draht, der im expandierten Zustand spiralförmig gewunden ist. Die Tragstruktur 11 kann durch Sputtern und/oder Ätzen hergestellt sein. Die Tragstruktur 11 kann auch als Gittergeflecht ausgebildet sein. Bevorzugte Materialien für das Innenohrimplantat 10 umfassen Nitinol, insbesondere gesputtertes, also durch ein PVD-Verfahren hergestelltes, Nitinol.

Das Nitinol bzw. allgemein eine Nickel-Titan-Legierung ist vorzugsweise elektrisch leitend und wird bevorzugt für die Bildung der Modulationsschichten 32, 33 eingesetzt. Es ist auch möglich, dass die tragende Schicht 30 eine Nickel-Titan-Legierung umfasst. Die Isolationsschichten 31, 34, 35 umfassen vorzugsweise ein Oxid.

Generell kann die Tragstruktur 11 derart ausgebildet sein, dass das Innenohrimplantat 10 in einen Zuführkatheter bzw. allgemein ein Zuführsystem wiedereinziehbar ist. Beispielsweise kann die Tragstruktur 11 an einem proximalen Ende konusförmig ausgebildet sein, wie beispielsweise in den Fig. 5b und 6b dargestellt. Insbesondere kann die Tragstruktur 11 am proximalen Ende mehrere Gitterstege 21 umfassen, die zu einem zentralen Punkt zusammenlaufen. Auf diese Weise ist ermöglicht, dass die Tragstruktur 11 bzw. allgemein das Innenohrimplantat 10 repositionierbar ist. Insbesondere kann das Innenohrimplantat 10 durch eine Relativbewegung zwischen einem Zuführsystem und der Tragstruktur 11 komprimierbar sein derart, dass die Position des Innenohrimplantats 10 korrigierbar ist. Es ist auch möglich, dass das Innenohrimplantat 10 in einer Weise mit dem Zuführsystem verbunden ist, dass eine Rotation des Innenohrimplantats 10 ermöglicht ist. Somit kann bei der Implantation eine gezielte Ausrichtung der Elektroden 12 erfolgen. Insbesondere kann das Innenohrimplantat 10 derart rotiert werden, dass die Elektroden 12 an der Basilarmembran 108 anliegen. Die Rotation des Zuführsystems kann zu einer Torsion des Innenohrimplantats 10 bzw. der Tragstruktur 11 führen. Vorzugsweise ist dazu ein Betätigungselement innerhalb des Zuführsystems vorgesehen, das mit dem proximalen Ende der Tragstruktur 11 verbunden oder verbindbar ist. Das Betätigungselement ermöglicht die axiale Relativbewegung zwischen der Tragstruktur 11 und dem Zuführkatheter und gegebenenfalls die Rotation der Tragstruktur 11. Das Betätigungselement kann fest mit der Tragstruktur 11 verbunden sein. Das Betätigungselement ist von der Tragstruktur 11 abkoppelbar, so dass die Tragstruktur 11 bzw. allgemein das Innenohrimplantat 10 vollständig implantierbar ist.

Bei den Ausführungsbeispielen gemäß Fig. 5a bis 6b sind die Elektroden 12 in axialer Richtung des Innenohrimplantats 10 auf einer Linie angeordnet bzw. erstrecken sich in Längsrichtung geradlinig entlang der Tragstruktur 11. Zusätzlich kann vorgesehen sein, dass mehrere Elektroden 12 in Umfangsrichtung benachbart angeordnet sind. Eine derartige Konfiguration ist beispielhaft in Fig. 7 dargestellt. Darin ist zu erkennen, dass insgesamt vier Reihen von Elektroden 12 vorgesehen sind, die sich jeweils in Längsrichtung des Innenohrimplantats 10 bzw. der Tragstruktur 11 erstrecken. Das hat den Vorteil, dass die Modulation, also die Erzeugung der elektrischen Felder, an den Verlauf des corti-Organs 109 angepasst werden kann. Beispielsweise können die Elektroden 12 einer in Umfangsrichtung benachbart angeordneten Elektrodengruppe aktiviert werden, die dem corti-Organ 109 im implantierten Zustand am nächsten liegt. Die Wahl der passenden Elektrode 12, also der Elektrode 12, die die beste Hörleistung für eine Schallfrequenz bewirkt, kann durch entsprechende Messmethoden ermittelt werden. Beispielsweise kann über die Messung der akustisch evozierten Potentiale ermittelt werden, welche der Elektroden 12, die in Umfangsrichtung benachbart angeordnet sind, bei Aktivierung den größten Nervenimpuls bewirkt.

Die Tragstruktur 11 kann im Allgemeinen durch ein kombiniertes PVD- und Ätzverfahren hergestellt werden. Auf diese Weise kann ein mehrschichtiger Aufbau der Tragstruktur 11 realisiert werden. Beispielsweise können in die Isolationsschicht 31 Fenster oder allgemein Öffnungen geätzt werden, um die darunter angeordnete Modulationsschicht, insbesondere die äußere Modulationsschicht 32 bereichsweise freizulegen. Der freigelegte Bereich der Modulationsschicht bildet eine Elektrode 12. Die Elektroden 12 können auch in die Tragstruktur 11 integriert werden, so dass die Elektroden 12 Teil der tragenden Struktur bzw. Tragstruktur 11 sind. Durch das vorgenannte Herstellungsverfahren können sowohl isolierende, als auch elektrisch leitende Schichten in einem Verfahrensschritt in die Tragstruktur 11 integriert werden bzw. selbsttragend die Tragstruktur 11 bilden. Fügeverfahren, beispielsweise zum Aufbringen der Elektroden 12 auf eine bestehende Tragstruktur 11, sind nicht erforderlich. Dies ermöglicht den Aufbau von komplexen Multielektrodenkonfigurationen innerhalb der Tragstruktur 11.

Der mehrschichtige Aufbau der Tragstruktur 11 ist gut in Fig. 8 erkennbar. Dabei zeigt Fig. 8 einen Querschnitt durch die Tragstruktur 11, wobei die Tragstruktur in einer Draufsicht analog zu den Ausführungsbeispielen gemäß Fig. 5a, 6a und 7 ausgebildet sein kann. Die Tragstruktur 11 weist einen mehrschichtigen Aufbau aus drei Schichten auf. Konkret ist vorgesehen, dass die Tragstruktur eine tragende Schicht 30 umfasst, auf der eine Isolationsschicht 31 angeordnet ist. Die tragende Schicht 30 kann mit der Isolationsschicht 31 überzogen sein. Auf der Isolationsschicht 31 ist eine äußere Modulationsschicht 32 aufgebracht. Die äußere Modulationsschicht 32 weist ein elektrisch leitendes Material auf. Die Isolationsschicht 31 weist hingegen ein elektrisch isolierendes Material auf. Die Isolationsschicht 31 isoliert die tragende Schicht 30 elektrisch von der äußeren Modulationsschicht 32. In Fig. 8 ist ferner die Basilarmembran 108 dargestellt, an der die äußere Modulationsschicht 32 aufliegt.

Die äußere Modulationsschicht 32 ist bei dem Ausführungsbeispiel gemäß Fig. 8 derart ausgebildet, dass die äußere Modulationsschicht 32 zumindest abschnittsweise über die Breite der Isolationsschicht 31 und der tragenden Schicht 30 übersteht. So können beispielsweise die plättchenförmigen Elektroden 12 gemäß Fig. 5a, 6a und 7 gebildet werden. Im Allgemeinen bildet die äußere Modulationsschicht 32 die Elektroden 12. Dabei ist in Fig. 8 gut zu erkennen, dass die äußere Modulationsschicht 32 stellenweise unterbrochen ist bzw. Lücken 36 aufweist, so dass die einzelnen Elektroden 12, die durch die äußere Modulationsschicht 32 gebildet sind, voneinander elektrisch isoliert sind. Die äußere Modulationsschicht 32 kann auch entlang der Gitterstege 21 unterbrochen sein bzw. Lücken 36 aufweisen, so dass nach außen die Isolationsschicht 31 freiliegt. Auf diese Weise trennt die Isolationsschicht 31 zwei separat aktivierbare Elektroden 12, die durch die äußere Modulationsschicht 32 gebildet sind.

Die plättchenförmigen oder allgemein verbreiterten Abschnitte der äußeren Modulationsschicht 32 bzw. generell die vergrößerten Elektroden 12 haben den Vorteil, dass die Stromleitungsfläche erhöht und somit die Stromdichte reduziert wird. Durch die niedrigere Stromdichte können Ströme mit kleinerer Stromspannung eingesetzt werden, wodurch das umliegende Körpergewebe geschont wird.

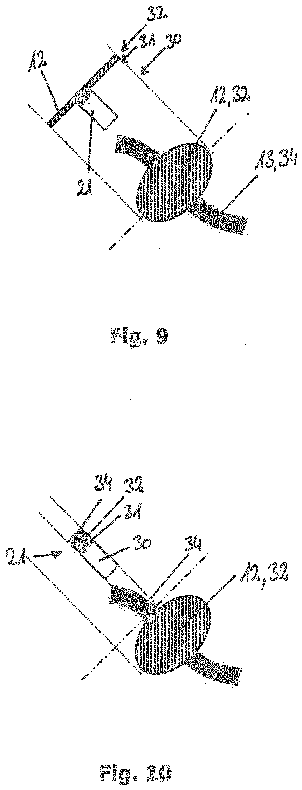

Vorzugsweise verläuft die äußere Modulationsschicht 32 über die gesamte Tragstruktur 11, so dass die äußere Modulationsschicht 32 auch eine Leitungsbahn 25 zur elektrischen Verbindung der Elektroden 12 mit einer Stromversorgung bildet. In den Bereichen, in denen die äußere Modulationsschicht 32 eine Leitungsbahn 25 für eine Elektrode 12 bildet, ist die äußere Modulationsschicht 32 vorzugsweise durch eine weitere Isolationsschicht 34 nach außen isoliert. Die äußere Modulationsschicht 32 ist also teilweise durch die weitere Isolationsschicht 34 bedeckt, wobei die bedeckten Abschnitte der Modulationsschicht 32 eine Leitungsbahn 25 und die unbedeckten bzw. freien Abschnitte der äußeren Modulationsschicht 32 eine Elektrode 12 bilden. In den Bereichen, in welchen die äußere Modulationsschicht 32 eine Leitungsbahn 25 bildet, weist die Modulationsschicht 32 im Wesentlichen bzw. hauptsächlich eine stromleitende Funktion auf. Dabei ist nicht ausgeschlossen, dass auch durch die Leitungsbahn 25 bzw. den die Leitungsbahn 25 bildenden Abschnitt der äußeren Modulationsschicht 32 eine Modulation erfolgt. Hauptsächlich erfolgt die Modulation jedoch in den nach außen nicht isolierten Bereichen der äußeren Modulationsschicht 32, also den Elektroden 12. Insbesondere liegt die äußere Modulationsschicht 32 im Bereich der Elektroden 12 nach außen hin frei, so dass ein elektrischer Kontakt zur Basilarmembran 108 bzw. allgemein zum Körpergewebe erreicht wird. Die freiliegende äußere Modulationsschicht 32 ist gut in Fig. 9 erkennbar, wobei ein Querschnitt durch eine Elektrode 12 der Tragstruktur 11 gezeigt ist. Im Querschnitt weist die Tragstruktur 11 im Bereich der Elektrode 12 den im Zusammenhang mit Fig. 8 erläuterten mehrschichtigen Aufbau auf.

In Fig. 10 ist eine Querschnittsdarstellung der Tragstruktur 11 gezeigt, wobei der Schnitt außerhalb der Elektrode 12 angeordnet ist, also durch eine Leitungsbahn 25 verläuft. Darin ist zu erkennen, dass die Modulationsschicht 32 durch eine weitere Isolationsschicht 34 bedeckt ist. Konkret kann die äußere Modulationsschicht 32 zwischen zwei Isolationsschichten 34 eingebettet sein. Die äußere Modulationsschicht 32 ist somit nach außen hin elektrisch isoliert. Die weitere Isolationsschicht 34 ist im Bereich der Elektrode 12 unterbrochen, so dass im Bereich der Elektrode 12 die äußere Modulationsschicht 32 freiliegt. Alternativ kann vorgesehen sein, dass die Tragstruktur 11 nur im Bereich der Elektroden 12 den mehrschichtigen Aufbau aus tragender Schicht 30, Isolationsschicht 31 und äußerer Modulationsschicht 32 aufweist. In den Bereichen außerhalb der Elektrode 12 kann die Tragstruktur 11 nur durch die tragende Schicht 30 gebildet sein. Andererseits kann in den Bereichen außerhalb der Elektroden 12 auch ein mehrschichtiger Aufbau der Tragstruktur 11 vorgesehen sein, wobei der mehrschichtige Aufbau nur die tragende Schicht 30 und die Isolationsschicht 31 umfasst. Die elektrische Verbindung zwischen den einzelnen Elektroden 12 und der Strom- bzw. Spannungsquelle kann dabei durch Kabel 14 erfolgen. Alternativ kann vorgesehen sein, dass die Elektroden 12 durch die Isolationsschicht 31 mit der tragenden Schicht 30 oder einer inneren Modulationsschicht 33 durchkontaktiert sind, wobei die innere Modulationsschicht 33 oder die tragende Schicht 30 in den Bereichen außerhalb der Elektroden 12 durch die Isolationsschicht 31 nach außen isoliert sind. Die innere Modulationsschicht 33 oder die tragende Schicht 30 bilden dabei die Leitungsbahnen 25 für die einzelnen Elektroden 12. Insbesondere kann die tragende Schicht 30 für einen Teil der Elektroden 12 als Leitungsbahn 25 genutzt werden. Dabei ist die tragende Schicht 30 von anderen Leitungsbahnen 25 isoliert, die zur Stromversorgung weiterer Elektroden 12 dienen. Auf diese Weise wird ein Kurzschluss vermieden.

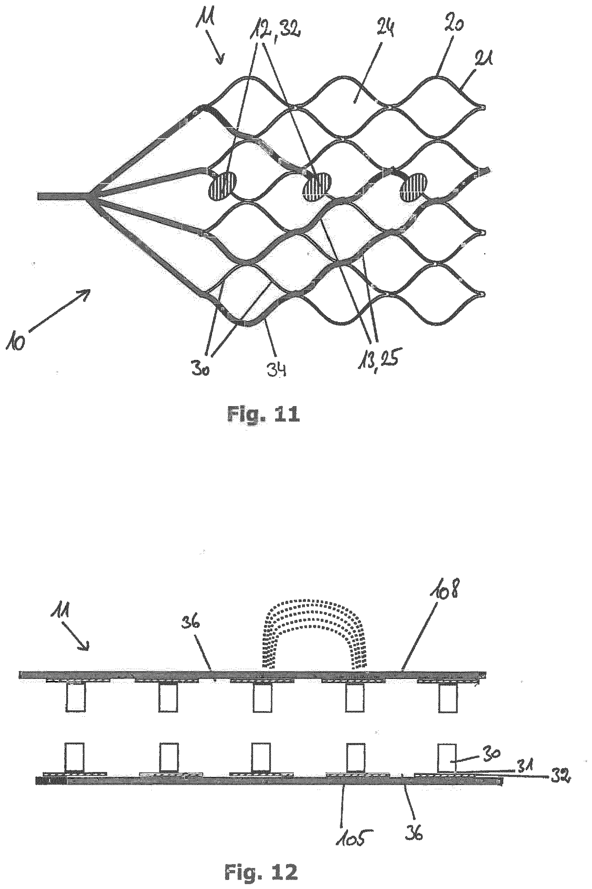

In Fig. 11 ist die Verbindung der Elektroden 12 durch Leitungsbahnen 25 mit der Stromversorgung (nicht dargestellt) gezeigt. Die Leitungsbahnen 25 verlaufen dabei entlang der Gitterstruktur 20. Die Leitungsbahnen 25 sind insbesondere durch die äußere Modulationsschicht 32 gebildet, die auch die Elektroden 12 bildet. Die Leitungsbahnen 25 sind durch eine weitere Isolationsschicht 34 nach außen isoliert bzw. abgedeckt. In den Bereichen zwischen den Elektroden 12 und den Leitungsbahnen 25 liegt die tragende Schicht 30 der Tragstruktur 11 frei. Die tragende Schicht 30 kann auch von einer Isolationsschicht überdeckt sein. Bei dem Ausführungsbeispiel gemäß Fig. 11 verlaufen also die Leitungsbahnen 25 im Wesentlichen seitlich an den jeweils weiter proximal angeordneten Elektroden 12 vorbei. Mit anderen Worten sind die Leitungsbahnen 25, die die weiter distal angeordneten Elektroden 12 mit der Stromversorgung verbinden, seitlich an den proximal angeordneten Elektroden 12 vorbeigeführt bzw. nutzen Gitterstege 21, die mit den proximalen Elektroden 12 nicht direkt verbunden sind.

Es kann vorgesehen sein, dass die Leitungsbahnen 25 auf derselben Ebene angeordnet sind, wie die Modulationsschicht 32, 33. Insbesondere können die Leitungsbahnen 25 durch die innere Modulationsschicht 33 oder äußere Modulationsschicht 32 gebildet sein. Dabei erfolgt die Trennung einzelner Leitungsbahnen 25 durch Unterbrechungen in der Modulationsschicht 32, 33. Beispielsweise kann die Modulationsschicht 32, 33 stellenweise durchgeätzt sein, so dass sich separate Leitungsbahnen 25 bilden. Eine Tragstruktur 11 mit einer äußeren Modulationsschicht 32, die zur Bildung mehrerer Leitungsbahnen 25 stellenweise unterbrochen ist, ist beispielsweise in Fig. 12 dargestellt.

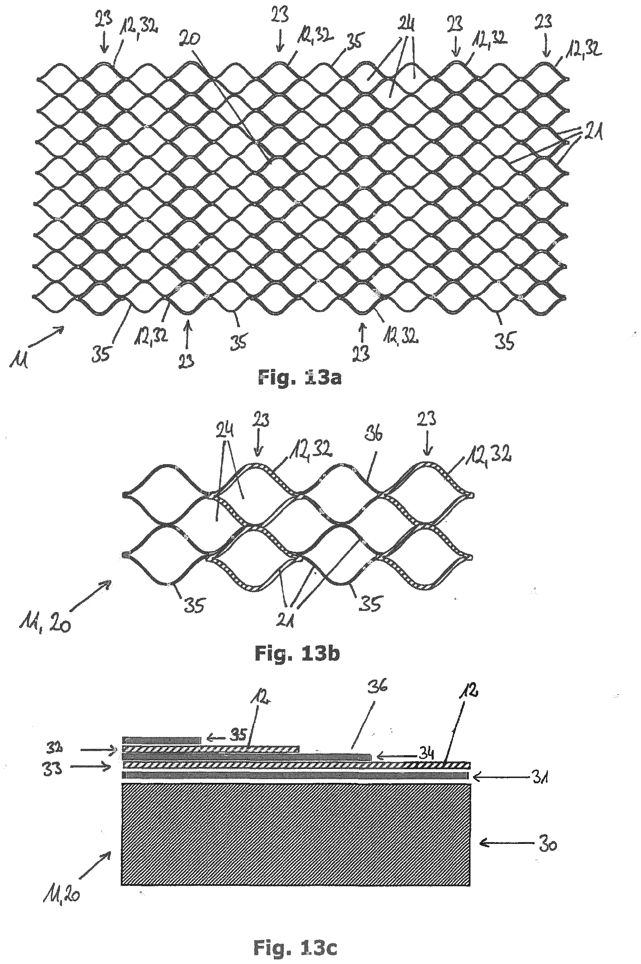

In den Fig. 13a bis 13c ist eine Variante dargestellt, wobei die Leitungsbahnen 25 in einem mehrschichtigen Aufbau der Tragstruktur 11 durch unterschiedliche Modulationsschichten 32, 33 gebildet sind. Fig. 13a zeigt den Aufbau der Tragstruktur 11, die als Gitterstruktur 20 mit Gitterstegen 21 ausgebildet ist. Die Gitterstruktur 20 umfasst mehrere in Längsrichtung benachbart und beabstandet angeordnete Elektroden 12, die insbesondere als Zellreihen 23 ausgebildet sind. Mit anderen Worten begrenzen die Gitterstege 21 jeweils Zellen, wobei jeweils eine Reihe von in Umfangsrichtung direkt benachbart angeordneter Zellen 24 jeweils eine Elektrode 12 bildet. In dem Detailausschnitt gemäß Fig. 13b ist gut zu erkennen, wie die einzelnen Elektroden 12, die jeweils durch eine Zellreihe 23 gebildet sind, in Längsrichtung der Gitterstruktur 20 bzw. der Tragstruktur 11 beabstandet angeordnet sind. Die einzelnen Zellreihen 23 bzw. Elektroden 12 sind voneinander elektrisch isoliert, so dass die Elektroden 12 separat aktivierbar sind. Es ist auch möglich, dass innerhalb der Zellreihen 23 mehrere in Umfangsrichtung voneinander isolierte Elektroden 12 vorgesehen sind. Insgesamt können über die Tragstruktur 11 mehrere Elektroden 12 musterartig angeordnet sein. Beispielsweise können sich die Elektroden 12 spiralförmig um die Längsachse der Tragstruktur 11 erstrecken. Im abgewickelten Zustand der Tragstruktur 11 verlaufen die Elektroden 12 dann schräg. Die Elektroden 12 können auch zickzackförmig versetzt angeordnet sein. Innerhalb einer Zellreihe 23 kann auch jede zweite oder jede dritte Zelle als Elektrode 12 ausgebildet sein. Die dazwischen liegenden Zellen stellen die elektrische Trennung, also die Isolation, der Elektroden 12 sicher. Andere Anordnungsmuster, insbesondere unregelmäßige Anordnungsmuster, sind möglich. Beispielsweise können in einer Zellreihe zwei Zellen als Elektroden 12 ausgebildet sein, wobei zwischen den Elektroden 12 eine Zelle als Isolationsbereich vorgesehen ist. Über den restlichen Umfang der Tragstruktur 11 weist die Zellreihe 23 elektrisch isolierte Zellen auf. Die Elektroden 12 jeder Zellreihe 23 können zu benachbarten Zellreihen 23 versetzt angeordnet sein.

Der mehrschichtige Aufbau der Gitterstruktur 20 gemäß Fig. 13a bzw. Fig. 13b ist in Fig. 13c als Längsschnitt dargestellt. Darin ist zu erkennen, dass die Tragstruktur 11 eine untere, tragende Schicht 30 umfasst, auf der eine Isolationsschicht 31 aufgebracht ist. Auf der Isolationsschicht 31 ist eine innere Modulationsschicht 33 angeordnet, die durch eine weitere Isolationsschicht 34 teilweise bedeckt ist. Die Isolationsschicht 31 und die innere Modulationsschicht 33 erstrecken sich vorzugsweise über die gesamte Oberfläche der tragenden Schicht 30. Zumindest bereichsweise ist die innere Modulationsschicht 33 durch die weitere Isolationsschicht 34 bedeckt. Über die weitere Isolationsschicht 34 erstreckt sich zumindest teilweise die äußere Modulationsschicht 32. Die äußere Modulationsschicht 32 ist wiederum teilweise durch eine äußere Isolationsschicht 35 bedeckt bzw. nach außen isoliert. Die weitere Isolationsschicht 34, die auf der inneren Modulationsschicht 33 aufgebracht ist, ist zumindest teilweise unterbrochen bzw. ausgespart, so dass die innere Modulationsschicht 33, wie in der linken Bildhälfte der Fig. 13c dargestellt, freiliegt. Die innere Modulationsschicht 33 bildet somit eine Elektrode 12 und gleichzeitig die zur Stromversorgung der Elektrode 12 genutzte Leitungsbahn. Die innere Modulationsschicht 33 ist mit der Stromversorgung verbunden, so dass die innere Modulationsschicht 33 einerseits eine Leitungsbahn 25 und andererseits eine mit der Leitungsbahn 25 verbundene Elektrode 12 bildet.

Die äußere Modulationsschicht 32 erstreckt sich über die weitere Modulationsschicht 34 und ist ebenfalls unterbrochen bzw. ausgespart oder endet vor dem Ende der weiteren Isolationsschicht 34 und bildet eine Lücke 36, so dass die weitere Isolationsschicht 34 zumindest bereichsweise freiliegt. Somit isoliert die weitere Isolationsschicht 34 die innere Modulationsschicht 33 einerseits gegenüber der Umgebung und andererseits gegenüber der äußeren Modulationsschicht 32. Die äußere Isolationsschicht 35, die die äußere Modulationsschicht 32 teilweise bedeckt, ist ebenfalls bereichsweise ausgespart, so dass die äußere Modulationsschicht 32 teilweise bzw. bereichsweise freiliegt. Analog zur inneren Modulationsschicht 33 bildet die äußere Modulationsschicht 32 somit eine Elektrode 12 des Innenohrimplantats 10 sowie gleichzeitig eine Leitungsbahn 25, die die Elektrode 12 mit einer Stromversorgung verbindet. Insgesamt bilden also die nach außen freiliegenden Bereiche bzw. Flächen der inneren Modulationsschicht 33 und der äußeren Modulationsschicht 32 jeweils die Elektroden 12, wobei die nach außen isolierten Bereiche der inneren Modulationsschicht 33 und der äußeren Modulationsschicht 32 die Leitungsbahnen 25 bilden. Die Isolationsschicht 34 kann auch mit einem Rand der äußeren Modulationsschicht 32 bündig abschließen. Mit anderen Worten kann die weitere Isolationsschicht 34 dieselbe Fläche bedecken, wie die äußere Modulationsschicht 32. In radialer Richtung der Tragstruktur 11 isoliert die weitere Isolationsschicht 34 die äußere Modulationssicht 32 von der inneren Modulationsschicht 33. In einer Draufsicht auf die Tragstruktur ist die weitere Isolationsschicht 34 nicht erkennbar, da sie durch die äußere Modulationsschicht 32 vollständig bedeckt ist. In der Draufsicht der Tragstruktur 11 wechseln sich somit die äußere Modulationsschicht 32 und die innere Modulationssicht 33 ab, wobei zwischen den Modulationsschichten 32, 33 keine weitere Isolationsschicht 34 erkennbar ist. Auf diese Weise kann die größtmögliche Außenoberfläche der Tragstruktur 11 zu Erzeugung elektrischer Felder genutzt werden.

Im weiteren Verlauf der Tragstruktur 11, also in Längsrichtung des Innenohrimplantats 10 baut sich der mehrschichtige Aufbau in umgekehrter Reihenfolge wieder auf. Das bedeutet, dass in Längsrichtung der Tragstruktur 11, also in einer nächsten Zellreihe 23, zunächst die Isolationsschicht 34 abschnittsweise die Außenoberfläche der Tragstruktur 11 bildet. Eine weitere, nachfolgende Zellreihe 23 ist wiederum als Elektrode 12 ausgebildet, wobei die Elektrode 12 durch die äußere Modulationsschicht 32 gebildet ist. In Längsrichtung der Tragstruktur 11 ist der Elektrode 12 eine weitere Zellreihe 23 nachgeordnet, die die äußere Isolationsschicht 35 umfasst, die dann die Außenfläche der Tragstruktur 11 bildet. Insgesamt sind also entlang der Tragstruktur 11 die weitere Isolationsschicht 34, die äußere Modulationsschicht 32 und die äußere Isolationsschicht 35 teilweise unterbrochen und bilden eine Lücke 36, wobei die Lücke 36 der weiteren Isolationsschicht 34 kleiner als die Lücke 36 der äußeren Modulationsschicht 32 ist, die wiederum kleiner als die Lücke 36 der äußeren Isolationsschicht 35 ist. Mit anderen Worten sind die weitere Isolationssicht 34, die äußere Modulationsschicht 32 und die äußere Isolationsschicht 35 stufenweise bzw. abgestuft unterbrochen, so dass nach außen isolierte und nach außen elektrisch leitende Abschnitte bzw. Bereiche oder Segmente der Tragstruktur 11 gebildet sind. Der mehrschichtige Aufbau der Tragstruktur 11 wird also zumindest für die oberen drei Schichten, also die weitere Isolationsschicht 34, die äußere Modulationsschicht 32 und die äußere Isolationsschicht 35, stufenweise abgebaut bzw. reduziert, bis die innere Modulationsschicht 33 freigelegt ist. Im weiteren Verlauf in Längsrichtung der Tragstruktur 11 wird der mehrschichtige Aufbau stufenweise wieder aufgebaut, bis die äußere Isolationssicht 35 die Außenfläche der Tragstruktur 11 bildet.

Bei der vorgenannten Konfiguration verläuft die Leitungsbahn 25, die weiter distal angeordnete Elektroden 12 mit der Stromversorgung koppelt, unter den proximal angeordneten Elektroden 12 bzw. Leitungsbahnen 25 hindurch. Auf diese Weise können durchgehende Zellreihen 23, die sich in Umfangsrichtung erstrecken, insbesondere über den gesamten Umfang der Tragstruktur 11 erstrecken, gebildet werden, die nicht durch Leitungsbahnen 25 zur Versorgung weiterer Zellreihen 23 unterbrochen sind. Die separate Aktivierbarkeit der einzelnen Elektroden 12 bzw. Zellreihen 23 ist dadurch gewährleistet, dass die Leitungsbahnen 25 in dem mehrschichtigen Aufbau der Tragstruktur 11 in unterschiedlichen Schichten angeordnet sind. Eine derartige Tragstruktur 11 ist einfach herstellbar, insbesondere durch entsprechende Abscheideverfahren, und ermöglicht eine besonders hohe Anzahl an unterschiedlich aktivierbaren Elektroden 12. Die Genauigkeit der Frequenzstimulation kann somit erhöht werden.

Eine Kombination seitlich vorbeilaufender Leitungsbahnen 25 mit einem mehrschichtigen Aufbau über- bzw. untereinander angeordneten Leitungsbahnen 25 ist möglich. Insbesondere können die einzelnen Modulationsschichten 32, 33 innerhalb der Schicht unterbrochen sein, so dass innerhalb einer einzigen Modulationsschicht 32, 33 mehrere Leitungsbahnen gebildet sind. Dies erhöht die Möglichkeiten für separat aktivierbare Elektroden.

Der zuvor beschriebene, mehrschichtige Aufbau der Tragstruktur 11 wird im Zusammenhang mit allen in der Anmeldung beschriebenen Ausführungsbeispielen beansprucht und offenbart.

Das Innenohrimplantat 10 der erfindungsgemäßen Hörprothese kann allgemein als Dauerimplantat ausgebildet sein. Das Innenohrimplantat 10 verbleibt also vorzugsweise langfristig im Körper. Dasselbe gilt in bevorzugter Weise für die Stromversorgung, insbesondere die vollimplantierbare Empfangsspule des TET-Systems. Insbesondere kann das Innenohrimplantat 10 von einem Zuführsystem völlig entkoppelbar sein.

Die Hörprothese kann ein transkutanes Energieübertragungssystem (TET-System) umfassen, so dass die für den Betrieb des Innenohrimplantats 10 verwendete Energie drahtlos und transkutan transferiert werden kann.

Es ist möglich, dass die Hörprothese für einen Kurzzeiteinsatz konzipiert ist, wobei das Innenohrimplantat 10 im Zuführsystem fest mit einem Betätigungselement verbunden ist. In diesem Fall kann die für den Betrieb des Innenohrimplantats 10 verwendete Energie außerhalb des Körpers bereitgestellt und über ein Kabel in das Innenohrimplantat 10 im Körperinneren übertragen werden. Das Innenohrimplantat 10 wird vorzugsweise in der cochlea implantiert und stimuliert die Haarzellen 110 bzw. Sensorzellen des corti-Organs 109. Das Innenohrimplantat 10 wird insbesondere bevorzugt in die scala tympani 105 eingeführt, in der die mechanischen akustischen Schwingungen durch die perilymphe an die Basilarmembran 108 übertragen werden. Das Innenohrimplantat 10 kann auch in die scala vestibuli eingesetzt werden.

Das TET-System umfasst vorzugsweise eine Senderspule, die außerhalb des Körpers angeordnet ist, und eine vollimplantierbare Empfangsspule, die die über die Senderspule eingekoppelte, elektrische Energie über Kabel oder allgemein Leitungen an das Innenohrimplantat 10 abgibt.

Die Funktionsweise der Hörprothese wird im Folgenden näher erläutert:
Durch eine Aktivierung einzelner Elektroden 12 des Innenohrimplantats 10, wie durch die Feldlinien in Fig. 3 angedeutet, können die Haarzellen 110 des corti-Organs 109 stimuliert werden. Durch eine geeignete Feedback-Funktion, die an sich bekannt ist, kann durch Signalmessung und eine entsprechende Regelung die Ansteuerung der Elektroden 12 automatisch oder aktiv durch den Arzt nach Untersuchung des Patienten geregelt werden. Durch die Möglichkeit, über die Tragstruktur unterschiedliche Elektroden mit einer Spannung zu beaufschlagen bzw. Signale zu erfassen, kann ein vergleichsweise breites Frequenzband genutzt werden. Im vorderen Bereich der cochlea 100, also in der Nähe des ovalen Fensters 102, werden die Elektroden 12 des Innenohrimplantats 10 bei akustischen Signalen mit hoher Frequenz aktiviert. Im inneren Bereich der cochlea 100, also im Bereich des apex, werden die Elektroden 12 bei akustischen Signalen mit niedriger Frequenz aktiviert. Eine andere aus medizinischer Sicht sinnvolle Zuordnung von Frequenzbereichen zu unterschiedlichen Elektroden 12 ist möglich.

Die Steuerung der Elektroden 12 erfolgt durch einen externen Sprachprozessor. Die Signalübertragung kann in an sich bekannter Weise transkutan durch magnetische Spulen bzw. allgemein ein transkutanes Energieübertragungssystem (TET-System) bewerkstelligt werden. Wenn der Sprachprozessor akustische Wellen beispielsweise mit niedriger Frequenz aufnimmt, werden die an entsprechenden Stellen des Innenohrimplantats 10 angeordneten Elektroden 12 mit Strom beaufschlagt. Durch die selektive Ansteuerbarkeit des Innenohrimplantats 10 bzw. der Elektroden 12 des Innenohrimplantats 10 wird die Qualität der Hörprothese verbessert. Der Sprachprozessor kann auch implantierbar sein.

Die Zuführung des Innenohrimplantats 10 erfolgt vorzugsweise durch ein Kathetersystem, wobei der Vorgang die folgenden Schritte umfasst:
In einem ersten Schritt wird ein Führungsdraht in die cochlea 100 eingeführt. Der Führungsdraht dient zur Führung eines Katheters, der über den Führungsdraht vorgeschoben wird, bis die Spitze des Katheters in der Nähe der apex der cochlea 100 angeordnet ist. In einem nächsten Schritt wird der Führungsdraht durch Zurückziehen durch den Katheter entfernt. Der Katheter verbleibt dabei in seiner Position. Ein weiterer Draht, insbesondere ein Transportdraht bzw. Pusher, der mit dem Innenohrimplantat 10 verbunden ist, wird durch den Katheter vorgeschoben. Auf diese Weise wird das Innenohrimplantat 10 durch den Katheter an den Bestimmungsort, nämlich die cochlea 100, gebracht. Sobald das Innenohrimplantat innerhalb des Katheters auf Höhe des Behandlungsortes, also auf der Höhe der endgültigen Implantationsstelle, sich befindet, wird der Katheter zurückgezogen, so dass das Innenohrimplantat freigelegt wird. Dabei expandiert das Innenohrimplantat 10, insbesondere die Tragstruktur 11. Durch weiteres Zurückziehen des Katheters wird das Innenohrimplantat 10 vollständig in der cochlea 100 freigesetzt, wobei das Innenohrimplantat 10 vollständig expandiert. Der Katheter wird weiter zurückgezogen, so dass auch die Kabel, die das Innenohrimplantat 10 später mit einer Stromversorgung koppeln, freigelegt werden. Wenn die Katheterspitze den Bereich bzw. den Ort erreicht hat, an dem die Stromversorgung, insbesondere die Empfangsspule des TET-Systems, implantiert wird, werden die Kabel vollständig freigelegt und das Innenohrimplantat 10 bzw. die Kabel von dem Transportdraht abgekoppelt. Die Kabel werden dann mit der Empfangsspule des TET-Systems elektrisch verbunden. Die Implantation ist damit abgeschlossen. Die Konfiguration bzw. Einstellung des Innenohrimplantats kann anschließend von außerhalb über ein entsprechendes Steuer- bzw. Programmiergerät erfolgen, das über die Sendespule und die Empfangsspule eine Datenverbindung zum Innenohrimplantat herstellt.

Zur Vereinfachung der Implantation kann das Innenohrimplantat 10 bereits bei der Herstellung in einen Katheter eingebracht werden. Ein Set umfassend einen Katheter mit einem darin angeordneten komprimierten Innenohrimplantat 10 wird im Rahmen der Anmeldung ebenfalls offenbart und beansprucht. Das Set kann gemeinsam abgepackt und sterilisiert sein, so dass sich die Handhabung des Sets während des operativen Eingriffs vereinfacht.

### Bezugszeichenliste

- 10: Innenohrimplantat
- 11: Tragstruktur
- 12: Elektroden
- 13: stromleitendes Element
- 14: Kabel
- 20: Gitterstruktur
- 21: Gittersteg
- 23: Zellreihe
- 24: Zelle
- 25: Leitungsbahn
- 30: tragende Schicht
- 31: Isolationsschicht
- 32: äußere Modulationsschicht
- 33: innere Modulationsschicht
- 34: weitere Isolationsschicht
- 35: äußere Isolationsschicht
- 36: Lücke
- 100: cochlea
- 101: Steigbügel
- 102: ovales Fenster
- 103: scala vestibuli
- 104: helicotrema
- 105: scala tympani
- 106: scala media
- 107: Reissner-Membran
- 108: Basilarmembran
- 109: corti-Organ
- 110: Haarzellen
- 111: nervus vestibulocochlearis
- 112: rundes Fenster

## Patentansprüche

1. Hörprothese mit einem Innenohrimplantat (10), das eine Tragstruktur (11) und wenigstens zwei in Längsrichtung voneinander beabstandet angeordnete Elektroden (12) umfasst, die getrennt voneinander aktivierbar und mit der Tragstruktur (11) verbunden sind, wobei die Tragstruktur (11) stentartig komprimierbar und expandierbar ist derart, dass die Tragstruktur (11) an die Form des Innenohrs anpassbar ist und wobei die Tragstruktur (11) einen mehrschichtigen Aufbau mit wenigstens einer elektrisch leitenden äußeren Modulationsschicht (32) und wenigstens einer elektrisch isolierten Isolationsschicht (34) aufweist, wobei die Modulationsschicht (32) wenigstens eine Elektrode (12) bildet und die Isolationsschicht (34) unterhalb der äußeren Modulationsschicht (32) angeordnet ist,
**dadurch gekennzeichnet, dass**
eine elektrisch leitende innere Modulationsschicht (33) vorgesehen ist, die durch die Isolationsschicht (34) von der äußeren Modulationsschicht (32) elektrisch isoliert ist, die zumindest bereichsweise eine Außenfläche der Tragstruktur (11) bildet, wobei die äußere Modulationsschicht (32) und die Isolationsschicht (34) bereichsweise unterbrochen sind derart, dass die innere Modulationsschicht (32) freiliegt, wobei die äußere Modulationsschicht (32) und die innere Modulationsschicht (33) zumindest in Längsrichtung abwechselnd angeordnet sind derart, dass die innere Modulationsschicht (33) und die äußere Modulationsschicht (32) jeweils wenigstens eine separat aktivierbare Elektrode (12) bilden.

2. Hörprothese nach Anspruch 1,
**dadurch gekennzeichnet, dass**
die Tragstruktur (11) eine Gitterstruktur (20) aus Gitterstegen (21) oder ein Gittergeflecht oder eine coilartige Spiralstruktur aufweist.

3. Hörprothese nach Anspruch 1 oder 2,
**dadurch gekennzeichnet, dass**
die Elektroden (12) stoffschlüssig mit der Tragstruktur (11) verbunden, insbesondere in die Tragstruktur (11) integriert, sind.

4. Hörprothese nach einem der vorhergehenden Ansprüche,
**dadurch gekennzeichnet, dass**
die äußere Modulationsschicht (32) zumindest bereichsweise eine Außenfläche der Tragstruktur (11) bildet und durch die Isolationsschicht (31) unterbrochen ist derart, dass die Isolationsschicht (31) und die äußere Modulationsschicht (32) zumindest in Längsrichtung abwechselnd angeordnet sind, wobei die durch die äußere Modulationsschicht (32) gebildeten Elektroden (12) durch die Isolationsschicht (31) voneinander elektrisch isoliert und separat aktivierbar sind.

5. Hörprothese nach einem der vorhergehenden Ansprüche,
**dadurch gekennzeichnet, dass**
die Elektroden (12) jeweils durch ein stromleitendes Element (13) mit einer Stromversorgung gekoppelt sind, wobei das stromleitende Element (13) ein Kabel (14), das entlang der Tragstruktur (11) geführt ist, oder eine Leitungsbahn (25) umfasst, die in die Tragstruktur (11) integriert ist.

6. Hörprothese nach Anspruch 5,
**dadurch gekennzeichnet, dass**
die Leitungsbahn (25) durch die äußere Modulationsschicht (32) oder die innere Modulationsschicht (33) gebildet ist.

7. Hörprothese nach Anspruch 5 oder 6,
**dadurch gekennzeichnet, dass**
die äußere Modulationsschicht (32) und/oder die innere Modulationsschicht (33) stellenweise unterbrochen sind derart, dass die äußere Modulationsschicht (32) und/oder die innere Modulationsschicht (33) jeweils mehrere Leitungsbahnen (25) und Elektroden (12) bilden, wobei die Elektroden (12) der äußeren Modulationsschicht (32) und die Elektroden (12) der inneren Modulationsschicht (33) jeweils separat aktivierbar sind.

8. Hörprothese nach einem der vorhergehenden Ansprüche,
**dadurch gekennzeichnet, dass**
das Innenohrimplantat (10) mit einer Steuereinheit, insbesondere einem Sprachprozessor, verbunden oder verbindbar ist derart, dass die Elektroden (12) zur Modulation elektrischer Felder variabel ansteuerbar sind.

9. Hörprothese nach Anspruch 8,
**dadurch gekennzeichnet, dass**
die Steuereinheit, insbesondere der Sprachprozessor, mit den Elektroden (12) signalverbunden ist derart, dass unterschiedliche Elektroden (12) in Abhängigkeit von der jeweiligen Schallfrequenz aktivierbar sind.

10. Hörprothese nach einem der vorhergehenden Ansprüche,
**dadurch gekennzeichnet, dass**
das Innenohrimplanat (10) mehrere Elektroden (12) umfasst, die einerseits in Längsrichtung und andererseits in Umfangsrichtung der Tragstruktur (11) angeordnet sind.

11. Hörprothese nach einem der vorhergehenden Ansprüche mit einem transkutanen Energie-Übertragungssystem.

## Claims

1. A hearing prosthesis having an inner ear implant (10) which comprises a support structure (11) and at least two electrodes (12) disposed so as to be separated from one another in the longitudinal direction, which can be activated separately from one another and which are connected to the support structure (11), wherein the support structure (11) can be compressed and expanded in the manner of a stent so that the support structure (11) can be adapted to the shape of the inner ear, and wherein the support structure (11) has a multi-layered construction with at least one electrically conductive outer modulation layer (32) and at least one electrically insulated insulation layer (34), wherein the modulation layer (32) forms at least one electrode (12) and the insulation layer (34) is disposed below the outer modulation layer (32),
**characterized in that**
an electrically conductive inner modulation layer (33) is provided which is electrically insulated from the outer modulation layer (32) by the insulation layer (34), which at least in regions forms an outer surface of the support structure (11), wherein the outer modulation layer (32) and the insulation layer (34) are interrupted in regions in a manner such that the inner modulation layer (32) is exposed, wherein the outer modulation layer (32) and the inner modulation layer (33) are arranged alternately at least in the longitudinal direction in a manner such that the inner modulation layer (33) and the outer modulation layer (32) respectively form at least one separately activatable electrode (12).

2. The hearing prosthesis as claimed in claim 1,
**characterized in that**
the support structure (11) has a mesh structure (20) formed from mesh webs (21) or a braided mesh or a coil-like spiral structure.

3. The hearing prosthesis as claimed in claim 1 or claim 2,
**characterized in that**
the electrodes (12) are mechanically bonded to the support structure (11), in particular integrated into the support structure (11).

4. The hearing prosthesis as claimed in one of the preceding claims,
**characterized in that**
the outer modulation layer (32) forms an outer surface of the support structure (11) at least in regions and is interrupted by the insulation layer (31) in a manner such that the insulation layer (31) and the outer modulation layer (32) are alternately arranged at least in the longitudinal direction, wherein the electrodes (12) formed by the outer modulation layer (32) are electrically insulated from each other by the insulation layer (31) and can be activated separately.

5. The hearing prosthesis as claimed in one of the preceding claims,
**characterized in that**
the electrodes (12) are respectively coupled to a power supply via an electrically conductive element (13), wherein the electrically conductive element (13) is a cable (14) which is guided along the support structure (11) or comprises a conductive pathway (25) which is integrated into the support structure (11).

6. The hearing prosthesis as claimed in claim 5,
**characterized in that**
the conductive pathway (25) is formed by the outer modulation layer (32) or the inner modulation layer (33).

7. The hearing prosthesis as claimed in claim 5 or claim 6,
**characterized in that**
the outer modulation layer (32) and/or the inner modulation layer (33) are interrupted in places in a manner such that the outer modulation layer (32) and/or the inner modulation layer (33) respectively form a plurality of conductive pathways (25) and electrodes (12), wherein the electrodes (12) of the outer modulation layer (32) and the electrodes (12) of the inner modulation layer (33) are respectively separately activatable.

8. The hearing prosthesis as claimed in one of the preceding claims,
**characterized in that**
the inner ear implant (10) is connected to or is connectable to a control unit, in particular a speech processor, in a manner such that the electrodes (12) can be controlled in a variable manner in order to modulate electrical fields.

9. The hearing prosthesis as claimed in claim 8,
**characterized in that**
the control unit, in particular the speech processor, is connected to the electrodes (12) for the purposes of signalling in a manner such that the various electrodes (12) can be activated as a function of the respective acoustic frequency.

10. The hearing prosthesis as claimed in one of the preceding claims,
**characterized in that**
the inner ear implant (10) comprises a plurality of electrodes (12) which on the one hand are disposed in the longitudinal direction and on the other hand are disposed in the circumferential direction of the support structure (11).

11. The hearing prosthesis as claimed in one of the preceding claims, having a transcutaneous energy transmission system.

## Revendications

1. Prothèse auditive comportant un implant d'oreille interne (10) qui comprend une structure porteuse (11) et au moins deux électrodes disposées espacées l'une de l'autre dans le sens longitudinal (12) et qui sont connectées de manière à être activables séparément l'une de l'autre et à la structure porteuse (11), la structure porteuse (11) pouvant être comprimée et expansée à la manière d'un stent, de sorte que la structure porteuse (11) est adaptable à la forme de l'oreille interne et la structure porteuse (11) présentant une structure multicouche comportant au moins une couche de modulation extérieure (32) conductrice d'électricité et au moins une couche d'isolation (34) isolant de l'électricité, la couche de modulation (32) constituant au moins une électrode (12) et la couche d'isolation (34) étant disposée en-dessous de la couche de modulation extérieure (32),
**caractérisée en ce**
**qu'**il est prévu une couche de modulation intérieure conductrice d'électricité (33) qui est isolée électriquement par la couche d'isolation (34) de la couche de modulation extérieure (32) qui constitue au moins par endroits une surface extérieure de la couche porteuse (11), la couche de modulation extérieure (32) et la couche d'isolation (34) étant interrompues par endroits de manière à ce que la couche de modulation intérieure (32) soit dégagée, la couche de modulation extérieure (32) et la couche de modulation intérieure (33) étant disposées alternativement du moins dans le sens longitudinal de manière à ce que la couche de modulation intérieure (33) et la couche de modulation extérieure (32) constituent respectivement une électrode activable séparément (12).

2. Prothèse auditive selon la revendication 1,
**caractérisée en ce que**
la structure porteuse (11) présente une structure grillagée (20) composée de traverses de grille (21) ou d'un réseau de grille ou d'une structure spiralée en forme de boucle.

3. Prothèse auditive selon la revendication 1 ou 2,
**caractérisée en ce que**
les électrodes (12) sont connectées par correspondance de matière à la structure porteuse (11), en particulier intégrées dans la structure porteuse (11).

4. Prothèse auditive selon une des revendications précédentes,
**caractérisée en ce que**
la couche de modulation extérieure (32) constitue du moins par endroits une surface extérieure de la structure porteuse (11) et est interrompue par la couche d'isolation (31), de sorte que la couche d'isolation (31) et la couche de modulation extérieure (32) sont disposées alternativement du moins dans le sens longitudinal, les électrodes (12) constituées par la couche de modulation extérieure (32) étant isolées électriquement l'une de l'autre par la couche d'isolation (31) et activables séparément.

5. Prothèse auditive selon une des revendications précédentes,
**caractérisée en ce que**
les électrodes (12) sont respectivement couplées par un élément conducteur d'électricité (13) à une alimentation électrique, l'élément conducteur d'électricité (13) comprenant un câble (14) qui est guidé le long de la structure porteuse (11), ou comprenant une bande conductrice (25) qui est intégrée dans la structure porteuse (11).

6. Prothèse auditive selon la revendication 5,
**caractérisée en ce que**
la bande conductrice (25) est constituée par la couche de modulation extérieure (32) ou la couche de modulation intérieure (33).

7. Prothèse auditive selon la revendication 5 ou 6,
**caractérisée en ce que**
la couche de modulation extérieure (32) et/ou la couche de modulation intérieure (33) sont interrompues par endroits, de sorte que la couche de modulation extérieure (32) et/ou la couche de modulation intérieure (33) constituent respectivement plusieurs bandes conductrices (25) et électrodes (12), les électrodes (12) de la couche de modulation extérieure (32) et les électrodes (12) de la couche de modulation intérieure (33) étant respectivement activables séparément.

8. Prothèse auditive selon une des revendications précédentes,
**caractérisée en ce que**
l'implant d'oreille interne (10) est connecté ou connectable à une unité de commande, en particulier un processeur vocal, de sorte que les électrodes (12) sont contrôlables de manière variable pour la modulation de champs électriques.

9. Prothèse auditive selon la revendication 8,
**caractérisée en ce que**
l'unité de commande, en particulier le processeur vocal, est connecté aux électrodes (12) du point de vue de la signalisation de manière à ce que différentes électrodes (12) soient activables en fonction de la fréquence acoustique respective.

10. Prothèse auditive selon une des revendications précédentes,
**caractérisée en ce que**
l'implant d'oreille interne (10) comprend plusieurs électrodes (12) qui sont disposées d'une part dans le sens longitudinal et d'autre part dans le sens circonférentiel de la structure porteuse (11).

11. Prothèse auditive selon une des revendications précédentes, comportant un système de transfert d'énergie transcutané.
